(19) Europäisches Patentamt
European Patent Office
Office européen des brevets

(11) EP 4 793 628 A1

(12) **EUROPEAN PATENT APPLICATION**
published in accordance with Art. 153(4) EPC

(43) Date of publication:
**19.08.2026 Bulletin 2026/34**

(21) Application number: **25887441.1**

(22) Date of filing: **15.10.2025**

(51) International Patent Classification (IPC):
***G01N 21/47*** *(2006.01)* ***G01N 33/543*** *(2006.01)*

(52) Cooperative Patent Classification (CPC):
**G01N 21/8483; G01N 21/47; G01N 33/543; G01N 33/54313; G01N 33/54388**

(86) International application number:
**PCT/JP2025/036374**

(87) International publication number:
**WO 2026/110529 (28.05.2026 Gazette 2026/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **20.11.2024 JP 2024202237**

(71) Applicants:
- **SymBio Pharmaceuticals Limited**
  **Tokyo 105-0001 (JP)**
- **NIPPON STEEL CHEMICAL & MATERIAL CO., LTD.**
  **Tokyo 103-0027 (JP)**

(72) Inventors:
- **MURAI Keiichi**
  **Tokyo 105-0001 (JP)**
- **HAYASHI Akio**
  **Tokyo 105-0001 (JP)**
- **ENOMOTO Yasushi**
  **Tokyo 103-0027 (JP)**
- **MATSUMURA Yasufumi**
  **Tokyo 103-0027 (JP)**

(74) Representative: **Eisenführ Speiser**
**Patentanwälte Rechtsanwälte PartGmbB**
**Gollierstraße 4**
**80339 München (DE)**

# (54) IMMUNOASSAY METHOD AND IMMUNOASSAY DEVICE

(57) To provide an immunoassay method and an immunoassay apparatus capable of quantifying even if a measurement target in a sample has a low concentration. An immunoassay method comprising a step of irradiating light onto a container or substrate containing a sample containing a measurement target and a plurality of labeling particles carrying an antibody capable of binding directly or indirectly to the measurement target, after contact between the sample containing the measurement target and the plurality of labeling particles carrying an antibody capable of binding directly or indirectly to the measurement target, wherein the light is irradiated into a region containing the labeling particles of the container or substrate; and a step of detecting light that is the irradiated light and has passed through the region containing the labeling particles of the container or substrate, wherein a wavelength of the irradiated light is a wavelength that generates Mie scattering by each of the plurality of labeling particles.

FIG.6

17
16
9
10
11
9
14

EP 4 793 628 A1

## Description

### Technical Field

The present invention relates to an immunoassay method and an immunoassay apparatus.

### Background Art

The immunoassay method (immunoassay) is a method of detecting or quantifying a trace component (antigen or antibody) as a measurement target contained in a sample, by utilizing a specific reaction between an antigen and an antibody. Since the antigen-antibody reaction has high sensitivity and selectivity of reaction, it is widely used in fields such as medicine, pharmaceuticals, health foods, biotechnology, and environment, for medicines and foods acting only on specific locations (chemical substances) in a living body, and in analytical devices and diagnostic agents detecting slight changes in a living body. There are various types of immunoassay methods depending on differences in measurement principles and the like. For example, enzyme immunoassay, radioimmunoassay, chemiluminescence immunoassay, fluorescence immunoassay, immunoradiometric assay (IRMA), immunoenzymometric assay (IEMA) labeling an antigen with an enzyme, latex agglutination method, latex agglutination inhibition method, immunochromatography method, ELISA method, Western blot method, hemagglutination method, hemagglutination inhibition method, and the like are known. All of these methods are common in that they detect or quantify a target component by utilizing a specific reaction between an antigen and an antibody.

Generally, the immunoassay method detects or quantifies a measurement target (antigen or antibody) by binding a labeling substance to any of an antibody, an antigen, or an antigen-antibody complex, and measuring a color development intensity of a color exhibited by the labeling substance, a signal intensity such as luminescence, fluorescence, or radiation emitted by the labeling substance, or turbidity of aggregates generated by cross-linking of labeling substances. As the labeling substance, particles generating color development such as gold colloid particles, nanoparticles, and quantum dots; substances generating signals such as radioactive elements, enzymes, chemiluminescent substances, and fluorescent dyes; and substances causing aggregation to occur such as latex and erythrocytes are known. Among these labeling substances, colored particles are widely used especially when one wishes to perform measurement in a short time, mainly in the immunochromatography method, because the presence or absence of a measurement target and the amount thereof can be easily determined visually or using a measurement device.

Further, Patent Literature 1 describes an immunoassay method using a lateral flow type test strip for chromatography containing, as a labeling substance, a resin-platinum complex in which a plurality of relatively small platinum particles are immobilized on a resin particle. It is described that this resin-platinum complex exhibits good color development and is excellent in durability and visibility.

Patent Literature 2 describes a chromatography measurement device that obtains a concentration of a measurement target substance in a solution to be inspected based on an optical signal obtained by developing the solution to be inspected on a chromatography test piece having at least three or more reagent immobilization parts arranged at intervals on a development layer, and detecting transmitted light or reflected light from the chromatography test piece obtained by irradiating light onto the chromatography test piece.

Patent Literature 3 describes an immunoanalysis method characterized by preparing an antibody or antigen labeled with noble metal colloid particles, adding a sample thereto to react an antigen or antibody contained in the sample with the antibody or antigen to form a noble metal colloid labeled immune complex, causing measurement light of a wavelength between visible and infrared to be incident on and totally reflected by a total reflection prism in a cell in which at least one surface is the total reflection prism, and measuring absorption in an absorption region specific to the noble metal colloid labeled immune complex among absorptions of the measurement light occurring at an interface between the total reflection prism and a sample mixed solution containing the labeled immune complex.

### Citation List

#### Patent Literatures

Patent Literature 1: Japanese Patent No. 6381642
Patent Literature 2: Japanese Unexamined Patent Application Publication No. 2006-250787
Patent Literature 3: Japanese Patent No. 3436982

### Summary of Invention

Problems to be solved by the invention

However, in all conventional immunoassay methods utilizing colored particles, the intensity of a color exhibited by a labeling substance (color development intensity) is measured visually or with an optical measurement device. When measuring color development intensity visually, although there is an advantage of being convenient without using an optical measurement device, there are personal errors in determination, and there is a risk that one cannot visually recognize a difference in color development intensity even if concentrations of measurement substances are different, and judges that they are the same concentration even if the concentrations are actually different. Since color development becomes weaker as a measurement target has a lower concentration, the risk that the above-mentioned accurate measurement becomes difficult increases. Furthermore, in the case of visual observation, there is also a problem in reproducibility because inspection results cannot be quantified.

On the other hand, when measuring color development intensity using an optical measurement device such as an absorptiometer, an absorption method of irradiating a light source onto a color development site by a labeling substance and measuring color development intensity of the reflected light thereof by a photodetector is common. However, there is a limit to absorbance per labeling particle, and particularly in a low concentration region, there is a problem that sensitivity is inferior because the number of labeling particles binding to a measurement target is small. Thus, in the conventional methods, the range of measurable concentration regions is limited, and it is difficult to detect or quantify a measurement target with high sensitivity especially in a low concentration region.

Further, although the immunochromatography optical measurement device described in Patent Literature 2 mentions obtaining a concentration of a measurement target substance in a solution to be inspected based on an optical signal obtained by detecting transmitted light or reflected light from a chromatography test piece (immunochromatography strip), it does not explain a measurement method by transmitted light in detail, and in the examples, it does not include constituent elements therefor. Also, Patent Literature 2 does not pay attention to a relationship between an irradiation light wavelength and a particle size of labeling particles, and much less mentions irradiating light under conditions generating Mie scattering. Furthermore, it does not even list being able to detect or quantify a measurement target even in a low concentration region as an issue. Similarly, Patent Literature 1 also does not pay attention to a relationship between an irradiation light wavelength and a particle size of labeling particles, and much less mentions irradiating light under conditions generating Mie scattering.

In Patent Literature 3, being able to detect or quantify a measurement target even in a low concentration region is not listed as an issue, but rather, it clearly describes that methods utilizing Rayleigh scattering or Mie scattering have a problem that detection sensitivity and measurement accuracy are low at low concentrations.

The present invention has been made in view of the above points, and an object thereof is to provide an immunoassay method and an immunoassay apparatus capable of detecting or quantifying a measurement target even in a low concentration region.

Means for solution of the problems

The present inventors have conducted intensive studies to solve the above problems, and have found that, instead of irradiating measurement light onto labeling particles and measuring color development intensity of the reflected light thereof, by irradiating measurement light onto labeling particles under conditions where Mie scattering occurs and detecting light intensity of the transmitted light thereof, it is possible to detect or quantify a measurement target of a lower concentration than conventionally possible.

The gist of the present invention is as follows.

[1] An immunoassay method comprising

a step of irradiating measurement light onto a container or substrate containing: a sample containing a measurement target; and a plurality of labeling particles carrying an antibody capable of binding directly or indirectly to the measurement target, after contact between the sample containing the measurement target and the plurality of labeling particles carrying an antibody capable of binding directly or indirectly to the measurement target, wherein the measurement light is irradiated into a region containing the labeling particles of the container or substrate; and

a step of detecting light (transmitted light) that has passed through the region among the irradiated measurement light,

wherein a wavelength of the irradiated measurement light is a wavelength that generates Mie scattering by each of the plurality of labeling particles.

[2] The immunoassay method according to [1], wherein the wavelength that generates Mie scattering by each of the

plurality of labeling particles is a wavelength satisfying $2<\alpha<10$ (where $\alpha=\pi\times$particle size of labeling particle (nm)/light wavelength (nm)).

[3] The immunoassay method according to [1] or [2], further having a step of separating, after the contact and before irradiating the measurement light, the antibody carried on a labeling particle that is bound directly or indirectly to the measurement target from the antibody carried on a labeling particle that is not bound to the measurement target.

[4] The immunoassay method according to any one of [1] to [3], further comprising a step of obtaining a concentration or amount of the measurement target from light intensity of the detected transmitted light.

[5] The immunoassay method according to any one of [1] to [4], wherein the step of irradiating the measurement light includes irradiating measurement light into the region containing the plurality of labeling particles of the container or substrate (hereinafter referred to as a first region), and irradiating measurement light into a second region outside the first region.

[6] The immunoassay method according to [5], comprising measuring a light intensity 1 of measurement light that has passed through the first region, and a light intensity 2 of measurement light that has passed through the second region.

[7] The immunoassay method according to [6], further comprising a step of determining a light attenuation rate by the following formula:

Light attenuation rate=(Light intensity 2-Light intensity 1)×100/Light intensity 2.

[8] The immunoassay method according to [7], further comprising a step of obtaining a calibration curve or an approximate line showing a relationship between the concentration or amount of the measurement target and the light attenuation rate, using a sample in which the concentration or amount of the measurement target is known in advance.

[9] The immunoassay method according to [7], further comprising a step of determining a light attenuation rate using a sample containing a measurement target of unknown concentration or amount, comparing the light attenuation rate with the calibration curve or approximate line obtained in advance in [8], and estimating the concentration or amount of the measurement target contained in the sample.

[10] The immunoassay method according to any one of [1] to [9], wherein the antibody capable of binding directly or indirectly to the measurement target is an antibody capable of specifically binding to the measurement target.

[11] The immunoassay method according to any one of [1] to [10], wherein the light is coherent measurement light.

[12] The immunoassay method according to [11], wherein the coherent measurement light is laser light.

[13] The immunoassay method according to any one of [1] to [12], wherein the labeling particles contain a metal.

[14] The immunoassay method according to any one of [1] to [13], wherein the labeling particles are metal-resin composite particles in which a plurality of metal particles are immobilized on a resin particle, or a plurality of metal particles are immobilized at least partially inside a resin particle.

[15] The immunoassay method according to [13] or [14], wherein the metal is silver, nickel, copper, gold, platinum, or palladium, or is an alloy containing any of these.

[16] The immunoassay method according to [13] or [14], wherein the metal contains platinum.

[17] The immunoassay method according to any one of [1] to [16], wherein the container or substrate is a well plate, a test strip, or a membrane.

[18] An immunoassay apparatus, having:

a holding part for holding a container or substrate containing a plurality of labeling particles carrying an antibody capable of binding directly or indirectly to a measurement target, inside or on the apparatus;

a light source disposed above the holding part for irradiating measurement light into a region containing the plurality of labeling particles of the container or substrate held by the holding part; and

a light detection part disposed on a side opposite to the light source across the container or substrate, detecting transmitted light irradiated from the light source and having passed through the region containing the labeling particles of the container or substrate,

wherein a wavelength of measurement light irradiated by the light source is a wavelength that generates Mie scattering by each of the plurality of labeling particles.

[19] The immunoassay apparatus according to [18], for use in the method according to any one of [1] to [17].

[20] The immunoassay apparatus according to [18] or [19], wherein the wavelength that generates Mie scattering by each of the plurality of labeling particles is a wavelength satisfying $2<\alpha<10$ (where $\alpha=\pi\times$particle size of labeling particle (nm)/light wavelength (nm)).

[21] The immunoassay apparatus according to any one of [18] to [20], further comprising the container or substrate comprising the plurality of labeling particles carrying an antibody capable of binding directly or indirectly to the measurement target.

[22] The immunoassay apparatus according to any one of [18] to [21], wherein the antibody capable of binding directly or indirectly to the measurement target is an antibody capable of specifically binding to the measurement target.
[23] The immunoassay apparatus according to any one of [18] to [22], further having a display part displaying light intensity of the transmitted light detected by the light detection part.
[24] The immunoassay apparatus according to any one of [18] to [23], further having an estimation part estimating an amount or concentration or amount of the measurement target contained in a sample based on light intensity of the transmitted light detected by the light detection part.
[25] The immunoassay apparatus according to any one of [18] to [24], wherein the immunoassay apparatus is configured to irradiate measurement light into each of the region containing the plurality of labeling particles of the container or substrate (first region) and a region outside the first region of the container or substrate (second region).
[26] The immunoassay apparatus according to [25], further having an estimation part estimating a concentration or amount of the measurement target contained in a sample based on a light intensity 1 when measurement light is irradiated into the first region and a light intensity 2 when measurement light is irradiated into the second region.
[27] The immunoassay apparatus according to any one of [18] to [26], wherein the light source is a light source irradiating coherent light.
[28] The immunoassay apparatus according to [27], wherein the coherent measurement light is laser light.
[29] The immunoassay apparatus according to any one of [18] to [28], wherein the light source is a light source capable of irradiating measurement light into a range that is within the region containing the labeling particles in the container or substrate and narrower than the region, when viewed from a light source side toward a direction of the light detection part.
[30] The immunoassay apparatus according to any one of [18] to [29], further having a slit part between the light source and the container or substrate, whereby the immunoassay apparatus is configured such that measurement light is irradiated from the light source into a range that is within a region containing the labeling particles in the container or substrate and narrower than the region, when viewed from a light source side toward a direction of the light detection part.
[31] The immunoassay apparatus according to any one of [18] to [30], further having a slit part between the container or substrate and the detection part, whereby the immunoassay apparatus is configured such that only measurement light irradiated into a region containing the labeling particles is detected by the light detection part.
[32] The immunoassay apparatus according to any one of [18] to [31], wherein the labeling particles contain a metal.
[33] The immunoassay apparatus according to any one of [18] to [32], wherein the labeling particles are metal-resin composite particles in which a plurality of metal particles are immobilized on a surface of a resin particle.
[34] The immunoassay apparatus according to [32] or [33], wherein the metal contains gold or platinum.
[35] The immunoassay apparatus according to [32] or [33], wherein the metal contains platinum.
[36] The immunoassay apparatus according to any one of [18] to [35], wherein the plurality of labeling particles exist overlapping three-dimensionally in the region when viewed from the light source toward a direction of the light detection part.
[37] The immunoassay apparatus according to any one of [18] to [36], wherein the container or substrate is a well plate, a test strip, or a membrane.
[38] The immunoassay apparatus according to any one of [18] to [37], for estimating a concentration or amount of the measurement target contained in a sample.
[39] A kit for immunoassay, comprising

the apparatus according to any one of [18] to [38]; and
a container or substrate containing a plurality of labeling particles carrying an antibody capable of binding directly or indirectly to a measurement target.

[40] A kit for immunoassay, comprising

the apparatus according to any one of [18] to [38]; and
a container or substrate capable of allowing disposition of a plurality of labeling particles carrying an antibody capable of binding directly or indirectly to a measurement target.

[41] The kit for immunoassay according to [40], further comprising a plurality of labeling particles carrying an antibody capable of binding directly or indirectly to a measurement target.
[42] A container or substrate containing a plurality of labeling particles carrying an antibody capable of binding directly or indirectly to a measurement target, for use in the method according to any one of [1] to [17], or for use in the apparatus according to any one of [18] to [38], or for use in the kit according to [39], or of use in the kit according to any one of [40] to [41].

[43] The container or substrate according to [42], which is a well plate, a test strip, or a membrane.
[44] The container or substrate according to [42], wherein the antibody capable of binding directly or indirectly to the measurement target is an antibody capable of specifically binding to the measurement target.
[45] The immunoassay method according to any one of [1] to [17], for use in an immunoassay using a labeling substance other than those used in a latex agglutination method.
[46] The immunoassay method according to any one of [1] to [17], for use in an immunoassay using a labeling substance other than those used in a latex agglutination method and a latex agglutination inhibition method.
[47] The immunoassay method according to [45] or [46], wherein the labeling substance used in the immunoassay using a labeling substance is replaced with the labeling particles used in the method according to any one of [1] to [17].
[48] The immunoassay method according to any one of [45] to [47], wherein irradiation of measurement light in the immunoassay using a labeling substance is performed by the method according to any one of [1] to [17].
[49] The immunoassay method according to any one of [45] to [48], wherein estimation of an amount or concentration of a measurement target in the immunoassay using a labeling substance is performed by the method according to any one of [1] to [17].
[50] The immunoassay method according to any one of [1] to [17], for use in an immunoassay selected from the group consisting of an immunochromatography method and an ELISA method.
[51] The immunoassay method according to [50], wherein the labeling substance used in the immunoassay selected from the group consisting of an immunochromatography method and an ELISA method is replaced with the labeling particles used in the method according to any one of [1] to [17].
[52] The immunoassay method according to [50] or [51], wherein irradiation of measurement light in the immunoassay selected from the group consisting of an immunochromatography method and an ELISA method is performed by the method according to any one of [1] to [17].
[53] The immunoassay method according to any one of [50] to [52], wherein estimation of an amount or concentration of a measurement target in the immunoassay selected from the group consisting of an immunochromatography method and an ELISA method is performed by the method according to any one of [1] to [17].
[54] The immunoassay method according to any one of [1] to [17], for use in an immunochromatography method.
[55] The immunoassay method according to [54], wherein the labeling substance used in the immunochromatography method is replaced with the labeling particles used in the method according to any one of [1] to [17].
[56] The immunoassay method according to [54] or [55], wherein irradiation of measurement light in the immunochromatography method is performed by the method according to any one of [1] to [17].
[57] The immunoassay method according to any one of [54] to [56], wherein estimation of an amount or concentration of a measurement target in the immunochromatography method is performed by the method according to any one of [1] to [17].
[58] The immunoassay method according to any one of [1] to [17], for use in a heterogeneous immunoassay using a labeling substance.
[59] The immunoassay method according to [58], wherein the labeling substance used in the heterogeneous immunoassay method using a labeling substance is replaced with the labeling particles used in the method according to any one of [1] to [17].
[60] The immunoassay method according to [58] or [59], wherein irradiation of measurement light in the heterogeneous immunoassay method using a labeling substance is performed by the method according to any one of [1] to [17].
[61] The immunoassay method according to any one of [58] to [60], wherein estimation of an amount or concentration of a measurement target in the heterogeneous immunoassay method using a labeling substance is performed by the method according to any one of [1] to [17].
[62] The immunoassay method according to any one of [1] to [17], for use in an immunoassay selected from a competitive heterogeneous labeling method or non-competitive heterogeneous labeling method using a labeling substance.
[63] The immunoassay method according to [62], wherein the labeling substance used in the immunoassay selected from a competitive heterogeneous labeling method or non-competitive heterogeneous labeling method using a labeling substance is replaced with the labeling particles used in the method according to any one of [1] to [17].
[64] The immunoassay method according to [62] or [63], wherein irradiation of measurement light in the immunoassay selected from a competitive heterogeneous labeling method or non-competitive heterogeneous labeling method using a labeling substance is performed by the method according to any one of [1] to [17].
[65] The immunoassay method according to any one of [62] to [64], wherein estimation of an amount or concentration of a measurement target in the immunoassay selected from a competitive heterogeneous labeling method or non-competitive heterogeneous labeling method using a labeling substance is performed by the method according to any one of [1] to [17].

Advantageous Effects of Invention

The immunoassay method and immunoassay apparatus of the present invention can detect or quantify a measurement target with high sensitivity even in a low concentration region.

Brief Description of Drawings

Fig. 1 is a schematic longitudinal cross-sectional view of a test strip for immunochromatography used in an embodiment of the present invention.
Fig. 2 is a schematic view when a housing case used for the test strip of Fig. 1 is viewed from a top surface side.
Fig. 3 is a schematic view when the housing case used for the test strip of Fig. 1 is viewed from a bottom surface side.
Fig. 4 is a schematic longitudinal cross-sectional view of a conventional test strip for immunochromatography.
Fig. 5 is a schematic longitudinal cross-sectional view of an immunoassay apparatus of an embodiment of the present invention.
Fig. 6 is an external view (perspective view) of an immunoassay apparatus of an embodiment of the present invention.
Fig. 7 is a schematic longitudinal cross-sectional view of an immunoassay apparatus of another embodiment of the present invention.
Fig. 8 is a graph showing a relationship between a dilution rate of a measurement target and a light attenuation rate in Example 1.
Fig. 9 is a graph showing a relationship between a dilution rate of a measurement target and a color development intensity in Comparative Examples 1 and 2.
Fig. 10 is a graph showing a relationship between a dilution rate of a measurement target and a light attenuation rate in Example 1 and Comparative Example 3.
Fig. 11 is a graph showing a relationship between a dilution rate of a measurement target and a light attenuation rate when light of various wavelengths was irradiated under conditions causing Mie scattering to occur.
Fig. 12 is a graph showing a relationship between a dilution rate of a measurement target and a light attenuation rate when labeling particles of various particle sizes were used under conditions causing Mie scattering to occur.

Description of Embodiments

<<Immunoassay Method>>

A first aspect of the present invention is an immunoassay method comprising

a step of irradiating measurement light onto a container or substrate containing: a sample containing a measurement target; and a plurality of labeling particles carrying an antibody capable of binding directly or indirectly to the measurement target, after contact between the sample containing the measurement target and the plurality of labeling particles carrying an antibody capable of binding directly or indirectly to the measurement target, wherein the measurement light is irradiated into a region containing the labeling particles of the container or substrate,
a step of detecting light (transmitted light) that has passed through the region among the irradiated measurement light, wherein a wavelength of the irradiated measurement light is a wavelength that generates Mie scattering by each of the plurality of labeling particles.

The method of the first aspect can be preferably used for detecting a measurement target contained in a sample, and/or for quantifying a measurement target (estimating a concentration or amount of a measurement target). Above all, it can be particularly preferably used for quantifying a measurement target (estimating a concentration or amount of a measurement target).
Hereinafter, each step and constituent element will be described.

<Step of Irradiating Measurement Light>

The method of the first aspect has a step of irradiating measurement light onto a container or substrate containing: a sample containing a measurement target; and a plurality of labeling particles carrying an antibody capable of binding directly or indirectly to the measurement target, after contact between the sample containing the measurement target and the plurality of labeling particles carrying an antibody capable of binding directly or indirectly to the measurement target. Here, the measurement light is irradiated into a region containing the labeling particles. Preferably, the measure-

ment light is irradiated into a region containing the measurement target labeled with the labeling particles. Here, the measurement target labeled with the labeling particles refers to a measurement target that binds directly or indirectly to an antibody carried by the labeling particle.

In the step of irradiating measurement light, measurement light is irradiated into a region containing a plurality of labeling particles of the container or substrate.

For example, in the case of the (lateral flow type) immunochromatography method, labeling particles that have labeled a measurement target are captured on a test line, and the width of measurement light irradiated at this time is preferably equal to or narrower than the width of the test line, and more preferably narrower than the width of the test line. By irradiating into a region containing labeling particles, it is possible to reduce noise, increase signal intensity, and further expect sensitivity improvement in a low concentration region.

Measurement light may be irradiated into a first region containing a plurality of labeling particles of the container or substrate, and into a second region different from the first region (preferably a region not containing a plurality of labeling particles), respectively. Irradiation of measurement light to the first region and the second region may be simultaneous, or may be at different timings. In the case of irradiating simultaneously, by preparing two light sources, it is possible to irradiate measurement light into the first region and into the second region simultaneously. It is also possible to irradiate measurement light from two light sources sequentially to irradiate measurement light into the first region and into the second region at different timings. Further, in the case of irradiating measurement light into the first region and into the second region at different timings, measurement light may be irradiated into the first region such that a light source comes above the first region, and subsequently the container or substrate is moved in a lateral direction (direction perpendicular to a direction from the light source toward the light detection part) until the light source comes above the second region to irradiate measurement light into the second region. Alternatively, measurement light may be irradiated into the first region such that a light source comes above the first region, and subsequently the light source is moved in a lateral direction (direction perpendicular to a direction from the light source toward the light detection part) until the light source comes above the second region to irradiate measurement light into the second region. Further, there is no particular restriction on the order of irradiating measurement light into the first region and into the second region, and measurement light may be irradiated to the second region after irradiating measurement light into the first region, or measurement light may be irradiated into the first region after irradiating measurement light into the second region. Examples of a means for moving the container or substrate include a slide device described later.

(Measurement Light)

Measurement light is light irradiated from a light source for the purpose of immunoassay (immune measurement).

The wavelength of the irradiated measurement light is a wavelength that generates Mie scattering by each of the plurality of labeling particles.

When measurement light hits a particle, it causes transmission, reflection, refraction, or scattering. Among these, scattering is divided into light blocking, diffraction, Rayleigh scattering, or Mie scattering depending on the wavelength of measurement light and the particle size of the particle irradiated with measurement light. It is known that when a particle size parameter $\alpha$ represented by $\alpha=\pi\times$particle size (nm)/light wavelength (nm) is $10^4<\alpha$, light blocking occurs; when $10<\alpha<10^4$, diffraction occurs; when $2<\alpha<10$, Mie scattering occurs; and when $\alpha<2$, Rayleigh scattering occurs (see, for example, Nikkan Kogyo Shimbun, Ltd., "Basic Physical Properties of Powder," edited by The Society of Powder Technology, Japan, page 25). Note that particle size is the diameter of a particle.

The wavelength of measurement light irradiated in the present invention is a wavelength that generates Mie scattering by each of the plurality of labeling particles. The wavelength that generates Mie scattering by each of the plurality of labeling particles is preferably a wavelength satisfying $2<\alpha<10$ (where $\alpha=\pi\times$particle size of labeling particle (nm)/light wavelength (nm)). Based on this formula, for example, when using labeling particles having a particle size of 450 nm as labeling particles, it is preferable to irradiate measurement light of a wavelength within a range of about 141.3 nm to about 706.5 nm as the light wavelength generating Mie scattering.

Further, the irradiated measurement light is preferably coherent light, and for example, more preferably light having coherent properties such as laser light. Since LED light is broad and its wavelength range and phase are not aligned, it has a characteristic of diverging. Therefore, scattering characteristics of light in a dispersion medium are also not constant, and penetrability of light is poor. Note that even an LED can be used if it has the above requirements. In contrast, coherent light (especially laser light) has aligned properties such as wavelength and phase, and the light has a characteristic of high directionality. By using coherent light (especially laser light), setting of a relationship between particle size determining particle parameters and wavelength of incident light becomes easy.

(Measurement Target)

The measurement target is not particularly limited, and various things can be used as the measurement target.

Further, the measurement target may be an antigen or may be an antibody. Examples thereof can include bacteria, fungi, viruses, nucleic acids, proteins, sugars, lipids, electrolytes, enzymes, hormones, tumor markers, drugs, antibodies produced in various living bodies, and the like as measurement targets.

The sample is not particularly limited as long as it can contain a measurement target, and various samples can be used. For example, the sample may be one generally used in clinical examination, and examples thereof include tissues, cells, cell extraction components, body fluids, and the like.

(Labeling particles Carrying Antibody Capable of Binding Directly or Indirectly to Measurement Target)

The labeling particles carrying an antibody capable of binding directly or indirectly to a measurement target may be labeling particles carrying an antibody capable of binding directly to the measurement target, or may be labeling particles carrying an antibody capable of binding indirectly to the measurement target via another substance.

That a labeling particle carries an antibody capable of binding directly to a measurement target means that the labeling particle is binding to an antibody that binds directly to the measurement target (for example, when the measurement target is an antigen, the antibody capable of binding to a substance that binds directly to the measurement target is a monoclonal antibody or polyclonal antibody specifically binding to the antigen; when the measurement target is an antibody (the said antibody is also an antigen), the antibody capable of binding to a substance that binds directly to the measurement target is a monoclonal antibody or polyclonal antibody binding to the said antibody).

That a labeling particle carries an antibody capable of binding indirectly to a measurement target means that the labeling particle is binding to an antibody capable of binding to a substance that binds directly to the measurement target (for example, when the substance that binds directly to the measurement target is a primary antibody, the antibody capable of binding to a substance that binds directly to the measurement target is a secondary antibody binding to the primary antibody).

Examples of labeling particles carrying an antibody capable of binding directly to a measurement target include labeling particles carrying an antibody capable of binding (specifically) to the antigen where the measurement target is an antigen. When the measurement target is an antibody, examples include labeling particles carrying an antibody capable of binding (specifically) to the antibody. As the antibody capable of binding (specifically) to the measurement target, a commercially available one may be used, or one manufactured by oneself may be used.

As the labeling particles carrying an antibody capable of binding indirectly to a measurement target via another substance, labeling particles carrying an antibody capable of binding to a substance capable of binding (directly or indirectly) to the measurement target can be used. For example, the antibody capable of binding indirectly to the measurement target via another substance may be a secondary antibody. In this case, by binding a secondary antibody carried on a labeling particle to an antibody (primary antibody) capable of binding directly to an antigen which is a measurement target, the measurement target can be labeled with the labeling particle.

For examples of the measurement target and the antibody capable of binding directly or indirectly to the measurement target, for example, descriptions of the direct method, indirect method, and sensitization method described in Item "II. Advantages and disadvantages of each technique of immunoassay" of DOJINDO Product Catalog "First Antibody Labeling Protocol-1" (URL) https://www.dojindo.co.jp/products/contents/inomu-antibody-enzyme-fluo-protein-fab-labeling-1.html can be referred to.

Therefore, in the method of the first aspect, the container or substrate contains a measurement target, a substance capable of specifically binding to said measurement target and not carried on a labeling particle, and a plurality of labeling particles carrying an antibody capable of binding indirectly to the measurement target (for example, a plurality of labeling particles carrying an antibody capable of binding to a substance capable of specifically binding to the measurement target), and by contact thereof, the antibody carried on said labeling particle may be indirectly binding to said measurement target via the substance specifically binding to said measurement target.

(Labeling particles)

In the method of the present invention, a plurality of labeling particles carrying an antibody capable of binding directly or indirectly to a measurement target are used.

A plurality of labeling particles carrying an antibody capable of binding directly or indirectly to a measurement target refers to a plurality of labeling particles in which each of the plurality of labeling particles carries an antibody capable of binding directly or indirectly to the measurement target. Each labeling particle may carry one antibody capable of binding directly or indirectly to the measurement target, or may carry a plurality of said antibodies. In the case of carrying a plurality, the plurality of said antibodies may be the same antibody or different antibodies.

"Carrying" means that an antibody capable of binding directly or indirectly to a measurement target is immobilized on a labeling particle, preferably on a surface of a labeling particle. There is no particular limitation on a method for immobilizing an antibody capable of binding directly or indirectly to a measurement target on the surface of a labeling

particle, and for example, the antibody capable of binding directly or indirectly to a measurement target can be immobilized on the surface of the labeling particle by a method by passive adsorption or a method by covalent bond via a cross-linking agent. Regarding the method by passive adsorption and the method by covalent bond via a cross-linking agent, for example, the following URL can be referred to (https://www.cosmobio.co.jp/support/technology/gold-nanoparticles-technical-note/covalent-conjugation-carboxylated-gold-nanoparticles-ctd.asp). Further, said antibody may be immobilized on the labeling particle via a linker.

The particle size of labeling particles is not particularly limited as long as it can generate Mie scattering when irradiated light hits each of the labeling particles, and labeling particles of various particle sizes can be used. Among them, labeling particles satisfying $2<\alpha<10$ (where $\alpha=\pi\times$particle size of labeling particle (nm)/light wavelength (nm)) are preferable. Based on this formula, for example, when irradiating light of a wavelength of 450 nm, the particle size of labeling particles is preferably within a range of about 286.6 nm to about 1433.1 nm (it is also preferably within a range of about 286.5 nm to about 1432.3 nm). Further, for example, when irradiated light is visible light (for example, a wavelength of about 360 nm to about 830 nm), the particle size of labeling particles is preferably within a range of about 229.3 nm to about 2643.3 nm (it is also preferably within a range of about 229.2 nm to about 2641.9 nm). Further, in the present invention, irradiated light is not limited to visible light, and for example, even with ultraviolet light or infrared light, a particle size within a range where Mie scattering occurs may be selected. The particle size (or average particle size) of labeling particles may be within a range of about 528.7 nm to about 3.2 mm when irradiating infrared rays (for example, a wavelength of about 830 nm to about 1 mm) (it may also be within a range of about 528.4 nm to about 3183.0 nm). When irradiating ultraviolet rays (about 10 nm to about 360 nm), it may be within a range of about 6.4 nm to about 1146.5 nm (it may also be within a range of about 6.4 nm to about 1145.9 nm). Note that labeling particles are preferably in an environment maintaining a uniform dispersion system in a medium to be measured. The average particle size can be measured using a centrifugal sedimentation particle size distribution analyzer, laser diffraction/scattering method, dynamic light scattering method, particle counter, field emission scanning electron microscope, or the like.

The average particle size of labeling particles can be obtained by area average diameter or the like, and is the diameter of the particle. For example, the average particle size of labeling particles can be a median diameter (d50) of a particle size distribution obtained by a centrifugal sedimentation particle size distribution analyzer.

The labeling particles are preferably particles through which irradiated measurement light does not permeate inside the labeling particle. If light does not permeate inside the labeling particle, light reaching the detector through the region containing the particles becomes light derived from Mie scattering, so sensitivity tends to increase. Examples of particles through which light does not permeate inside the labeling particle preferably include labeling particles containing a metal, and additionally resin particles that do not transmit light, particles using silica, and the like.

Examples of resin particles include polyamine, polyamide, polypeptide, polyurethane, polystyrene, polyurea, polyimide, polyimidazole, polyoxazole, polypyrrole, polyaniline, and the like. Furthermore, polyamines such as poly-2-vinylpyridine, poly-3-vinylpyridine, and poly-4-vinylpyridine, acrylic resins, phenol resins, epoxy resins, cellulose resins, melamine resins, and the like can be widely used.

The labeling particles preferably contain a metal, and more preferably contain a metal on at least a part or the whole of the surface of the labeling particle. By the labeling particles containing a metal, particles through which irradiated measurement light does not permeate inside the labeling particle are easily obtained. The labeling particles may be particles composed of metal, may be particles in which metal is coated on the surface of particles other than metal, or may be particles in which a plurality of metal particles are immobilized on and/or inside particles other than metal. Among these, the labeling particles are preferably particles in which metal is coated on the surface of particles other than metal, particles in which a plurality of metal particles are immobilized on particles other than metal, or particles in which a plurality of metal particles are immobilized at least partially inside particles other than metal, and more preferably particles in which a plurality of metal particles are immobilized on particles other than metal or particles in which a plurality of metal particles are immobilized at least partially inside particles other than metal, and still more preferably metal-resin composite particles in which a plurality of metal particles are immobilized on resin particles or metal-resin composite particles in which a plurality of metal particles are immobilized at least partially inside particles.

That a plurality of metal particles are immobilized at least partially inside particles other than metal means that a plurality of metal particles are immobilized at least partially inside particles other than metal (preferably, a part of the plurality of metal particles is exposed to the outside of the particles other than metal, and the rest exists in a state of being included inside the particles other than metal). That a plurality of metal particles are immobilized at least partially inside resin particles means that a plurality of metal particles are immobilized at least partially inside resin particles (preferably, a part of the plurality of metal particles is exposed to the outside of the resin particles, and the rest exists in a state of being included inside the resin particles).

Examples of metal-resin composite particles in which a plurality of metal particles are immobilized on resin particles and a production method thereof include metal-resin composite particles and a production method thereof described in International Publication WO2017/010391.

In addition, examples of metal-resin composite particles include a structure in which a resin is coated on the

surface of metal particles, and a structure in which a metal layer (which may be an aggregate of metal particles) is laminated on the surface of resin particles.

Further, there is no particular limitation on the type of metal, and various metals can be used. Examples thereof include metals such as platinum, gold, iron, iron oxide, nickel, silver, copper, or palladium, or alloys containing one or more of these. Further, examples thereof include metals such as platinum, gold, iron, iron oxide, nickel, silver, copper, or palladium, or alloys containing any of these. Among these, the labeling particles preferably contain at least one metal selected from the group consisting of platinum, gold, and palladium, more preferably contain at least one metal selected from the group consisting of platinum and gold, and still more preferably contain platinum.

Further, the metal is preferably a metal such as platinum, gold, iron, iron oxide, nickel, silver, copper, or palladium, or an alloy containing one or more of these. Further, the metal is preferably a metal such as platinum, gold, iron, iron oxide, nickel, silver, copper, or palladium, or an alloy containing any of these, more preferably at least one metal selected from the group consisting of platinum and gold, and still more preferably platinum.

When using particles in which a plurality of metal particles are immobilized on the surface of particles other than metal, there is no particular limitation on the average particle size of the plurality of metal particles, but for example, the average particle size may be within a range of 0.1 to 50 nm, may be within a range of 0.5 to 30 nm, may be within a range of 1 to 20 nm, may be within a range of 1 to 10 nm, or may be within a range of 2 to 8 nm.

Regarding the average particle size of metal particles, for example, an area average diameter of 100 randomly selected metal particles (an average value of area diameters of each of 100 metal particles) is measured from an image observed by a field emission scanning electron microscope (FE-SEM; manufactured by Hitachi High-Technologies Corporation, SU-9000), and this area average diameter can be defined as the average particle size of metal particles.

When using particles in which a plurality of metal particles are immobilized on particles other than metal as labeling particles, there is no particular limitation on the ratio of metal immobilized on particles other than metal, but for example, the ratio of metal in labeling particles may be within a range of 10 to 80% by mass, may be within a range of 10 to 60% by mass, may be within a range of 20 to 50% by mass, or may be within a range of 30 to 50% by mass.

(Container or Substrate)

The container or substrate used in the method of the first aspect contains: a sample containing a measurement target; and a plurality of labeling particles carrying an antibody capable of binding directly or indirectly to the measurement target, wherein the sample containing the measurement target and the plurality of labeling particles carrying an antibody capable of binding directly or indirectly to the measurement target are in contact. Measurement light is irradiated onto the container or substrate where the sample containing the measurement target has been contacted with the labeling particles.

There is no limitation on the type of container or substrate, and various containers or substrates can be used. However, it is a condition that the container or substrate is configured such that when measurement light is irradiated from a light source into a region containing labeling particles in the container or substrate (and a region not containing labeling particles), the light can pass through the region containing labeling particles in the container or substrate (or each of said region and other regions (preferably regions not containing labeling particles)) and reach a photodetector. By this, improvement in sensitivity can be expected.

For example, in the container or substrate, at least a portion through which measurement light passes may be composed of a transparent member or may be composed of a porous material so that measurement light can pass through. Examples of porous materials include porous membranes such as nitrocellulose membranes, woven fabrics, non-woven fabrics, and the like. Further, when using a substrate in a form accommodated in a container, the container accommodating the substrate may have a cavity in at least a portion through which light passes among its bottom part (that is, there may be a hole in the light transmitting portion of the container).

Examples of the container or substrate can include well plates such as 96-well plates, test strips (for immunochromatography), membranes, specimen tubes, test tubes, glass slides, and the like. Further, the test strip or membrane may be one accommodated in a housing case. Among these, as the container or substrate, well plates such as 96-well plates, test strips such as test strips for immunochromatography, membranes for test strips, and the like can be preferably used.

In the well plate, the bottom of the well plate is preferably transparent so that light can pass through the well plate when light is irradiated from above.

In the test strip, at least a portion through which light passes among a backing sheet is preferably removed so that light can pass through when light is irradiated from above. Further, when the test strip or membrane is accommodated in a housing case, at least a portion through which light passes among the front surface and back surface of the housing case is preferably removed (opened).

The container or substrate after contact between the sample containing the measurement target and the plurality of labeling particles carrying an antibody capable of binding directly or indirectly to the measurement target is a container or

substrate after bringing the sample containing the measurement target into contact with the plurality of labeling particles carrying an antibody capable of binding directly or indirectly to the measurement target (after causing an antigen-antibody reaction to occur). Since the measurement target is labeled with the labeling particles by the antigen-antibody reaction, by irradiating light into a region where labeling particles exist and measuring light intensity of the transmitted light thereof, it is possible to detect the presence of the measurement target or estimate the concentration or amount of the measurement target.

There is no particular limitation on a method for bringing a sample containing a measurement target into contact with a plurality of labeling particles carrying an antibody capable of binding directly or indirectly to the measurement target. For example, in the case of the immunochromatography method, a plurality of labeling particles carrying an antibody capable of binding directly or indirectly to a measurement target can be arranged in a conjugate pad 4 of a test strip 1, and a (liquid) sample containing the measurement target can be added dropwise into a sample pad 3. After dropwise addition, the sample develops from the sample pad 3 to the conjugate pad 4, the sample containing the measurement target contacts the plurality of labeling particles carrying an antibody capable of binding directly or indirectly to the measurement target, and the measurement target is labeled with the labeling particles. Thereafter, the sample containing the measurement target particles labeled with the labeling particles develops to a membrane 5 and passes through a test line 6, but since an antibody capable of binding to the measurement target is immobilized on the test line 6, the measurement target labeled with the labeling particles is captured at the test line 6. This test line 6 is a region where the measurement target labeled with the labeling particles exists, and by irradiating light into this region, it is possible to measure light intensity of transmitted light of the light irradiated into the region where the measurement target labeled with the labeling particles exists.

Note that other substances not captured at the test line 6 develop together with the sample to a control line 7. Since an antibody capable of binding to the antibody capable of binding directly or indirectly to the measurement target is immobilized on the control line 7, a plurality of labeling particles carrying an antibody capable of binding directly or indirectly to the measurement target, which did not bind to the measurement target, are captured at the control line 7. The remaining sample will subsequently be absorbed by an absorption pad 8.

(Region Where Measurement Target Labeled with Labeling particles Exists)

After contact between the sample containing the measurement target and the plurality of labeling particles carrying an antibody capable of binding directly or indirectly to the measurement target, the labeling particles that have labeled the measurement target may move to a desired region as necessary. After contact between the sample containing the measurement target and the plurality of labeling particles carrying an antibody capable of binding directly or indirectly to the measurement target, the labeling particles that have labeled the measurement target exist in or move to a region where the measurement target labeled with the labeling particles exists. Measurement light can be irradiated onto the region where the labeling particles that have labeled the measurement target exist (after the labeling particles that have labeled the measurement target have moved as necessary). The region where the measurement target labeled with the labeling particles exists preferably has a thickness in a direction from the light source to the detector.

As the thickness of the region increases, labeling particles exist over that thickness, so light hits more labeling particles before reaching the photodetector. Since the attenuation amount of light increases (attenuates) exponentially according to the thickness (according to the frequency of hitting labeling particles), the adjustment of the thickness makes it easier to measure the concentration or amount of the measurement target with high sensitivity. For example, in the case of the immunochromatography method, since the measurement target labeled with labeling particles exists in the membrane of the test line, the thickness of said region corresponds to the thickness of the membrane of the test line. Also, in the case of the ELISA method, since the measurement target labeled with labeling particles exists in the liquid in each well of the well plate, the thickness of said region corresponds to the thickness of the liquid (or the thickness of a region where the measurement target labeled with labeling particles exists in the liquid). Since the region where the measurement target labeled with said labeling particles exists is a region transmitting light irradiated from the light source side to the detector side, it may be called a medium. Similarly, the thickness of said region may be called the thickness of the medium. In this case, there is no particular limitation on the type of medium, and various media can be used. Examples thereof include membranes of test strips used in the immunochromatography method and the like, liquids (developing solvents, buffers, water, organic solvents) contained in wells of well plates used in the ELISA method and the like, gels and thin layers (silica particle gels, ceramic particles, resin particles, cellulose particles), woven fabrics, non-woven fabrics, paper, and the like.

As reference, when light travels through a thickness of a homogeneous semitransparent medium, the attenuation amount is exponential, and is represented by the following Lambert-Beer law.

$$F_{B}(\lambda) = F_{A}(\lambda) \cdot \exp[-\alpha(\lambda) \cdot x]$$

(In the formula, $F_A(\lambda)$ is spectral radiant flux (unit is [W/nm]), x is thickness, $F_B(\lambda)$ is spectral radiant flux at a position advanced by x (unit is [W/nm]), and $\alpha(\lambda)$ is a spectral absorption coefficient of the medium)

There is no particular limitation on the thickness of the region where the measurement target exists or the thickness of the medium, and an optimal thickness may be appropriately used based on the type of measurement target and its expected concentration.

<Separation Step>

The method of the first aspect may further have a step of separating, after contact between the sample containing the measurement target and the plurality of labeling particles carrying an antibody capable of binding directly or indirectly to the measurement target, and before irradiating measurement light, the antibody carried on a labeling particle that is bound directly or indirectly to the measurement target from the antibody carried on a labeling particle that is not bound to the measurement target.

By including the separation step, only the antibody carried on a labeling particle that is bound directly or indirectly to the measurement target is easily included in the region containing labeling particles onto which measurement light is irradiated, and sensitivity further improves.

In the separation step, the antibody carried on the labeling particle binding directly or indirectly to the measurement target and the antibody carried on the labeling particle not binding to the measurement target may be separated (automatically) by configuring such that, like a lateral flow type immunochromatographic assay, while the sample and/or labeling particles flow through a test strip, only the antibody carried on the labeling particle binding directly or indirectly to the measurement target is captured in a predetermined area, and the antibody carried on the labeling particle not binding to the measurement target is not captured in the above area. Alternatively, both can be separated manually by providing an area for capturing the antibody carried on the labeling particle binding directly or indirectly to the measurement target on the bottom surface of a container, and discarding a liquid containing the antibody carried on the labeling particle not binding to the measurement target.

<Step of Detecting Light>

The method of the first aspect includes a step of detecting transmitted light that has passed through the region containing labeling particles of the container or substrate among measurement light. There is no particular limitation on means for detecting transmitted light, and transmitted light may be detected using, for example, a photodiode, a photomultiplier tube, a photoconductive element, an imaging element, or the like.

Note that in the step of irradiating measurement light, when measurement light is irradiated into a first region containing a plurality of labeling particles (preferably measurement target labeled with labeling particles) of the container or substrate, and into a second region outside the first region, respectively, the step of detecting transmitted light detects not only transmitted light that has passed through the first region but also transmitted light that has passed through the second region.

Note that in this specification, light irradiated from a light source toward a container or substrate is called measurement light, and light that has passed through the container or substrate among measurement light is called transmitted light.

<Step of Detecting Measurement Target/Estimating Concentration or Amount of Measurement Target>

The method of the first aspect of the present invention may include a step of detecting a measurement target from light intensity of detected light, and may further include a step of estimating a concentration or amount (preferably concentration) of a measurement target from light intensity of detected light.

Note that for light intensity, a value (for example, lux) obtained by an illuminometer using a photodiode or the like is used.

Further, when estimating the concentration or amount of a measurement target, light may be irradiated into a first region containing a plurality of labeling particles of the container or substrate and into a second region different from the first region (preferably a region not containing a plurality of labeling particles), respectively, and a light intensity 1 of light that has passed through the first region and a light intensity 2 of light that has passed through the second region may be measured. Further, the method of the first aspect of the present invention may further have a step of determining a light attenuation rate by the following formula.

$$\text{Light attenuation rate} = (\text{Light intensity 2} - \text{Light intensity 1}) \times 100 / \text{Light intensity 2}$$

The light attenuation rate is an index indicating how much the light intensity of transmitted light when light is irradiated into the first region containing the measurement target labeled with a plurality of labeling particles is reduced relative to the light intensity of transmitted light when light is irradiated into the second region, and allows grasping the degree of attenuation of transmitted light caused by Mie scattering.

<Step of Obtaining Calibration Curve>

The method of the first aspect may further include a step of obtaining a calibration curve showing a relationship between the concentration or amount of the measurement target and the light attenuation rate (or a relationship between the dilution rate of the measurement target and the light attenuation rate) using a sample in which the concentration or amount of the measurement target is known in advance. The calibration curve is preferably linear, and more preferably an approximate line. For the calibration curve or approximate line, for example, by plotting values obtained above with the vertical axis (y) being light attenuation rate (%) and the horizontal axis (x) being dilution rate, and with both axes as a logarithmic graph of $Log_{10}$ scale, a calibration curve or approximate line can be obtained by a regression equation (y=ax+b) of simple regression analysis by the least squares method. By obtaining a calibration curve, it is possible to execute the method of the first aspect using a sample containing a measurement target of unknown concentration or amount to calculate its light attenuation rate, compare the calculated light attenuation rate with the calibration curve, and estimate the concentration or amount of the measurement target.

The dilution rate indicates a ratio of a measurement substance added to another substance when diluting the measurement substance with another substance (for example, a liquid such as water). For example, when a measurement substance of X mg/mL is prepared and diluted 10000 times with a liquid, the dilution rate is 1/10000 ($1\times10^{-4}$), and the concentration of the measurement substance after dilution in this case is $X\times10^{-4}$ mg/mL. Similarly, when a measurement substance of X g/mL is prepared and diluted 40000 times with a liquid, the dilution rate is 1/40000 ($2.5\times10^{-5}$), and the concentration of the measurement substance after dilution in this case is $2.5X\times10^{-5}$ mg/mL.

<Step of Estimating Concentration or Amount of Measurement Target>

The method of the first aspect may further include a step of comparing a light attenuation rate obtained using a sample of unknown concentration or amount of a measurement target with a calibration curve obtained in advance, and estimating the concentration or amount of the measurement target contained in the sample of unknown concentration or amount of the measurement target.

The calibration curve has the concentration or amount (dilution rate) of the measurement target on the horizontal axis and the light attenuation rate on the vertical axis. Therefore, the light attenuation rate obtained using a sample of unknown concentration or amount of a measurement target is compared with the calibration curve to obtain the concentration or amount of the measurement target in the calibration curve corresponding to the light attenuation rate obtained using the sample of unknown concentration or amount of the measurement target. The obtained concentration or amount can be estimated to be the concentration or amount of the measurement target in the sample.

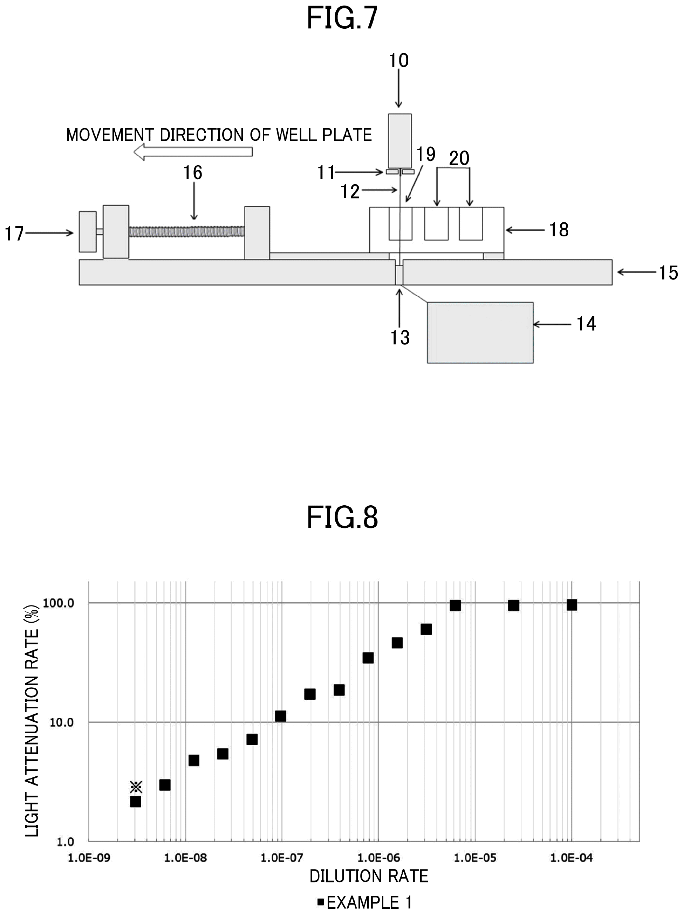

The following is an example of estimating the concentration of a measurement target using the data in Fig. 8. First, a measurement target of 3.12 mg/mL is diluted to various concentrations with a solvent (Tables 1 and 2), the light attenuation rate at each concentration is obtained (Fig. 8), and a calibration curve showing the relationship between the dilution rate of the measurement target and the light attenuation rate is obtained. Next, using a measurement target of unknown concentration, a light attenuation rate is obtained by the method of the first aspect. Using this light attenuation rate and the calibration curve, a dilution rate corresponding to the light attenuation rate can be obtained. A value obtained by multiplying the obtained dilution rate by 3.12 mg/mL, which is the concentration of the measurement target before dilution, can be estimated to be the concentration of the measurement target of unknown concentration.

Preferably, the method of the first aspect may be an immunoassay method comprising

(A) a step of irradiating measurement light onto a container or substrate containing a sample containing a measurement target of which a concentration or amount is known (hereinafter referred to as sample 1) and a plurality of labeling particles carrying an antibody capable of binding directly or indirectly to the measurement target, after contact between the sample 1 containing the measurement target and the plurality of labeling particles carrying an antibody capable of binding directly or indirectly to the measurement target, wherein the measurement light is irradiated into each of a region containing the labeling particles of the container or substrate (hereinafter referred to as a first region) and a second region outside the first region;
(B) a step of detecting light having passed through the first region (transmitted light 1) and light having passed through the second region (transmitted light 2), respectively;
(C) a step of determining a light attenuation rate from a light intensity 1 of the detected transmitted light 1 and a light intensity 2 of the transmitted light 2 by the following formula:

Light attenuation rate=(Light intensity 2-Light intensity 1)×100/Light intensity 2;

(D) a step of obtaining a calibration curve showing a relationship between the concentration or amount of the measurement target and the light attenuation rate (or a relationship between the dilution rate of the measurement target and the light attenuation rate);
(E) a step of irradiating measurement light onto a container or substrate containing a sample containing a measurement target of unknown concentration or amount (hereinafter referred to as sample 2) and a plurality of labeling particles carrying an antibody capable of binding directly or indirectly to the measurement target, after contact between the sample 2 containing the measurement target and the plurality of labeling particles carrying an antibody capable of binding directly or indirectly to the measurement target, wherein the measurement light is irradiated into each of a region containing the labeling particles of the container or substrate (hereinafter referred to as a first region) and a second region outside the first region;
(F) a step of detecting light having passed through the first region (transmitted light 1) and light having passed through the second region (transmitted light 2), respectively;
(G) a step of determining a light attenuation rate from a light intensity 1 of the detected transmitted light 1 and a light intensity 2 of the transmitted light 2 by the following formula:

Light attenuation rate=(Light intensity 2-Light intensity 1)×100/Light intensity 2;

(H) a step of obtaining a concentration or amount of the measurement target (or a dilution rate of the measurement target) corresponding to the light attenuation rate obtained in the step (G), using the light attenuation rate obtained in the step (G) and the calibration curve obtained in the step (D);
(I) as an optional step, a step of estimating the concentration or amount of the measurement target in the sample 2 from the dilution rate obtained in the step (H),

wherein a wavelength of the irradiated measurement light is a wavelength that generates Mie scattering by each of the plurality of labeling particles.

In this case, the wavelength that generates Mie scattering by each of the plurality of labeling particles is preferably a wavelength satisfying $2<\alpha<10$ (where $\alpha=\pi\times$particle size of labeling particle (nm)/light wavelength (nm)).

As the first region and the second region, those described above can be used. The first region is preferably a region containing a measurement target labeled with labeling particles. The second region is preferably a region not containing a measurement target labeled with labeling particles, and more preferably a region not containing labeling particles.

Further, the above method may have, after the contact and before irradiating measurement light, a step of separating the antibody carried on a labeling particle that is bound directly or indirectly to the measurement target from the antibody carried on a labeling particle that is not bound to the measurement target.

As a method for creating a calibration curve showing the relationship between the concentration or amount of the measurement target and the light attenuation rate (or the relationship between the dilution rate of the measurement target and the light attenuation rate) using sample 1 in which the concentration or amount of the measurement target is known, for example, the method described above can be used.

<<Immunoassay Apparatus>>

The apparatus of the second aspect of the present invention is an immunoassay apparatus, having:

a holding part for holding a container or substrate comprising a plurality of labeling particles carrying an antibody capable of binding directly or indirectly to a measurement target;
a light source disposed above the holding part for irradiating light into a region containing the plurality of labeling particles of the container or substrate held by the holding part; and
a light detection part disposed on a side opposite to the light source across the container or substrate, detecting light irradiated from the light source and having passed through the region containing the labeling particles of the container or substrate,
wherein a wavelength of light irradiated by the light source is a wavelength that generates Mie scattering by each of the plurality of labeling particles.

The apparatus of the second aspect can be preferably used for detection of a measurement target contained in a sample, and estimation of a concentration or amount of a measurement target.

Further, the apparatus of the second aspect can be suitably used for the method of the first aspect. Therefore, the apparatus of the second aspect is preferably an apparatus for use in the method of the first aspect.

As the measurement target, the antibody capable of binding directly or indirectly to the measurement target, the labeling particles, and the like, those listed in the first aspect can be used.

(Light Source)

The apparatus of the second aspect has a light source which is disposed above the holding part and is for irradiating light into a region containing a plurality of labeling particles of the container or substrate held by the holding part.

The measurement light irradiated from the light source preferably has a wavelength that generates Mie scattering by each of the plurality of labeling particles, and more preferably has a wavelength satisfying $2<\alpha<10$ (where $\alpha=\pi\times$particle size of labeling particle (nm)/light wavelength (nm)).

Further, there is no particular limitation on the type of light source, but it is preferably a light source generating a light amount capable of withstanding exponential light amount attenuation due to Mie scattering while having coherent characteristics, and more preferably laser light. Note that measurement light other than laser light can be used as long as it has the above properties.

Further, the light source is preferably a light source capable of irradiating light into a range that is the same as or narrower than a region containing labeling particles in the container or substrate (preferably a region containing a measurement target labeled with labeling particles) when viewed from the light source side toward the direction of the light detection part, and more preferably a light source capable of irradiating light into a range narrower than the region containing labeling particles in the container or substrate.

Further, the apparatus of the second aspect is preferably configured to irradiate light into each of a first region containing the plurality of labeling particles of the container or substrate and a second region not containing the plurality of labeling particles of the container or substrate. Irradiation of light to the first region and the second region may be simultaneous, or may be at different timings. In the case of irradiating simultaneously, by preparing two light sources, it is possible to irradiate light into the first region and into the second region simultaneously. In this case, it is preferable to prepare two light detection parts as well. That is, it is preferable that light source A irradiates light into the first region and light detection part A detects the transmitted light thereof, and light source B irradiates light into the second region and light detection part B detects the transmitted light thereof. In the case of irradiating light into the first region and into the second region at different timings, light may be irradiated into the first region such that a light source comes above the first region, and subsequently the container or substrate is moved in a lateral direction (direction perpendicular to a direction from the light source toward the light detection part) until the light source comes above the second region to irradiate measurement light into the second region. Alternatively, light may be irradiated into the first region such that a light source comes above the first region, and subsequently the light source is moved in a lateral direction (direction perpendicular to a direction from the light source toward the light detection part) until the light source comes above the second region to irradiate measurement light into the second region. Further, there is no particular limitation on the order of irradiating light into the first region and into the second region, and light may be irradiated into the second region after irradiating light into the first region, or light may be irradiated into the first region after irradiating light into the second region. Examples of a means for moving the container or substrate include a slide device described later.

(Slit Part)

The apparatus of the second aspect may have a slit part between the light source and the container or substrate. By this, when viewed from the light source side toward the direction of the light detection part, it becomes easier to irradiate light into a range narrower than the region containing labeling particles in the container or substrate. The slit part is one in which a small slit hole is provided in a plate-like member, and light from the light source passes through the slit hole, whereby light can be irradiated into a narrower range. There is no particular limitation on the shape of the slit hole, and it may be circular, square, rectangular, triangular, or other shapes, and a slit hole of an appropriate shape may be appropriately selected according to the shape of the region containing labeling particles. The maximum width of the slit hole is preferably smaller than the width of light irradiated from the light source to the slit.

Further, the apparatus of the second aspect may have a slit part between the container or substrate and the detection part, instead of the slit part between the light source and the container or substrate, or in addition to the slit part. By this, it becomes easy for the light detection part to detect only light irradiated into the region containing labeling particles. There is no particular limitation on the shape of the slit hole, and it may be circular, square, rectangular, triangular, or other shapes, and a slit hole of an appropriate shape may be appropriately selected according to the shape of the region containing labeling particles. The maximum width of the slit hole is preferably smaller than the width of light irradiated from

the light source into the region containing labeling particles and transmitted to the slit.

(Holding Part)

The apparatus of the second aspect has a holding part for holding a container or substrate containing a plurality of labeling particles carrying an antibody capable of binding directly or indirectly to a measurement target. There is no particular limitation on the shape of the holding part as long as it can hold the container or substrate; for example, the immunoassay apparatus may hold the container or substrate by having a holding part in a shape in which a concave shape (or U-shape) extends in one direction, and accommodating the container or substrate in a concave space formed by the concave shape (or U-shape) extending in one direction. By having a holding part in a shape in which a concave shape (or U-shape) extends in one direction, it becomes easier to slide the container or substrate in either the front-rear direction or the left-right direction. Alternatively, the immunoassay apparatus may have a holding part of a mechanism that sandwiches and fixes the container or substrate. In this case, it is preferable that the holding part has a function of moving the container or substrate in the front-rear direction and/or the left-right direction.

(Container or Substrate)

The apparatus of the second aspect may further contain a container or substrate containing a plurality of labeling particles carrying an antibody capable of binding directly or indirectly to a measurement target.

Further, the apparatus of the second aspect may further contain a container or substrate. Further, the container or substrate may be prepared separately from the apparatus, and held by the holding part in the apparatus after bringing a sample containing a measurement target into contact with a plurality of labeling particles carrying an antibody capable of binding directly or indirectly to the measurement target inside the container or substrate. For example, in the case of the ELISA method, since the plurality of labeling particles carrying an antibody capable of binding directly or indirectly to a measurement target are not contained in a container in advance but are added during a test, the apparatus of the second aspect may further contain the container or substrate in a form not containing the plurality of labeling particles carrying an antibody capable of binding directly or indirectly to a measurement target.

As examples of the container or substrate, those listed in the first aspect can be used.

The container or substrate is preferably such that when light is irradiated from the light source, a plurality of labeling particles exist overlapping in a region containing the plurality of labeling particles when viewed from the light source toward the direction of the light detection part. That a plurality of labeling particles exist overlapping means that a plurality of labeling particles exist densely in any of the X-axis direction (left-right direction), Y-axis direction (front-rear direction), and Z-axis direction (thickness direction) in said region of the container or substrate, and when viewed from the light source toward the direction of the light detection part, the plurality of labeling particles appear to overlap each other.

(Light Detection Part)

The apparatus of the second aspect has a light detection part which is disposed on a side opposite to the light source across the container or substrate and detects light irradiated from the light source and having passed through the region containing labeling particles of the container or substrate. The light detection part may be composed of, for example, a photodiode or the like.

Note that when the light source irradiates light into a first region containing a plurality of labeling particles of the container or substrate and into a second region different from the first region (preferably a region not containing a plurality of labeling particles), respectively, the light detection part detects not only light that has passed through the first region but also light that has passed through the second region.

(Display Part)

The apparatus of the second aspect may further have a display part displaying light intensity of light detected by the light detection part or the like. The display part is connected to the light detection part by wire or wirelessly, and displays the light intensity detected by the light detection part, the light attenuation rate calculated from the light intensity, and/or the concentration or amount of the measurement target estimated from the light intensity or light attenuation rate on the display part.

(Estimation Part)

The apparatus of the second aspect may further have an estimation part estimating an amount or concentration or amount of a measurement target contained in a sample based on light intensity of light detected by the light detection

part. The estimation part may be incorporated inside the display part, or may exist separately from the display part.

Further, the apparatus of the second aspect is configured to irradiate light into each of a first region containing a plurality of labeling particles of the container or substrate and a second region not containing the plurality of labeling particles of the container or substrate, and may further have an estimation part estimating a concentration or amount of a measurement target contained in a sample based on a light intensity 1 when light is irradiated into the first region and a light intensity 2 when light is irradiated into the second region. As a method for estimating the concentration or amount of a measurement target, the method described in the first aspect can be used.

(Slide Part)

The apparatus of the second aspect may further have a slide part for sliding the container or substrate. The slide part may slide the container or substrate so as to push it out far away when viewed from the slide part, may slide the container or substrate so as to pull it toward the slide part, or may have both mechanisms.

Further, the slide part may be one capable of sliding the container or substrate only in the left-right direction, or may be one capable of sliding it not only in the left-right direction but also in the front-rear direction. By being able to slide the container or substrate in the left-right direction and the front-rear direction, for example, even when there are multiple wells in both front-rear and left-right directions like a 96-well plate, it is possible to move the well plate by the slide part and continuously measure light intensity of measurement targets existing in the plurality of wells.

<<Kit for Immunoassay 1>>

The kit of the third aspect of the present invention is a kit for immunoassay comprising the apparatus of the second aspect, and a container or substrate containing a plurality of labeling particles carrying an antibody capable of binding directly or indirectly to a measurement target.

As the measurement target, the antibody capable of binding directly or indirectly to the measurement target, the labeling particles, the container or substrate, and the like, those described in the first aspect or the second aspect can be used.

The kit of the third aspect can be preferably used in the method of the first aspect.

<<Kit for Immunoassay 2>>

The kit of the fourth aspect of the present invention is a kit for immunoassay comprising the apparatus of the second aspect, and a container or substrate capable of allowing disposition of a plurality of labeling particles carrying an antibody capable of binding directly or indirectly to a measurement target.

The container or substrate capable of allowing disposition of a plurality of labeling particles is preferably a container or substrate capable of allowing disposition of a plurality of labeling particles in the container or substrate or on the container or substrate.

The kit of the fourth aspect preferably further comprises a plurality of labeling particles carrying an antibody capable of binding directly or indirectly to a measurement target.

As the measurement target, the antibody capable of binding directly or indirectly to the measurement target, the labeling particles, the container or substrate, and the like, those described in the first aspect or the second aspect can be used.

The kit of the fourth aspect can be preferably used in the method of the first aspect.

<<Container or Substrate for Immunoassay>>

The container or substrate of the fifth aspect of the present invention is a container or substrate containing a plurality of labeling particles carrying an antibody capable of binding directly or indirectly to a measurement target, for use in the method of the first aspect, or for use in the apparatus of the second aspect, or for use in the kit of the third aspect, or for use in the kit of the fourth aspect.

As the measurement target, the antibody capable of binding directly or indirectly to the measurement target, the labeling particles, the container or substrate, and the like, those described in the first aspect or the second aspect can be used.

<Application to Various Immunoassay Methods>

The method, apparatus, kit, and container or substrate of the present invention can be applied to various types of immunoassay methods. Examples of immunoassay methods to which the method of the present invention can be applied

include, for example, enzyme immunoassay, radioimmunoassay, chemiluminescence immunoassay, fluorescence immunoassay, immunoradiometric assay (IRMA), immunoenzymometric assay (IEMA) labeling an antigen with an enzyme, latex agglutination method, latex agglutination inhibition method, immunochromatography method, ELISA method, Western blot method, hemagglutination method, hemagglutination inhibition method, and the like.

The method of the present invention is preferably for use in an immunoassay using a labeling substance other than those used in a latex agglutination method, and more preferably for use in an immunoassay using a labeling substance other than those used in a latex agglutination method and a latex agglutination inhibition method. The labeling substance used in the immunoassay using a labeling substance is preferably replaced with the labeling particles used in the method of the first aspect. In the immunoassay using a labeling substance, for example, when using an antibody containing a labeling substance (as an example, a radioisotope or an enzyme) or an antigen containing a labeling substance, the labeling substance among them may be replaced with the labeling particles used in the method of the first aspect. Further, irradiation of measurement light in the immunoassay using a labeling substance is preferably performed by the method of the first aspect. Further, estimation of an amount or concentration of a measurement target in the immunoassay using a labeling substance is preferably performed by the method of the first aspect.

The method of the present invention is preferably for use in an immunoassay selected from the group consisting of an immunochromatography method and an ELISA method. The labeling substance used in the immunoassay selected from the group consisting of an immunochromatography method and an ELISA method is preferably replaced with the labeling particles used in the method of the first aspect. Irradiation of measurement light in the immunoassay selected from the group consisting of an immunochromatography method and an ELISA method is preferably performed by the method of the first aspect. Estimation of an amount or concentration of a measurement target in the immunoassay selected from the group consisting of an immunochromatography method and an ELISA method is preferably performed by the method of the first aspect.

The method of the present invention is particularly preferably for use in an immunochromatography method. The labeling substance used in the immunochromatography method is preferably replaced with the labeling particles used in the method of the first aspect. Irradiation of measurement light in the immunochromatography method is preferably performed by the method of the first aspect. Estimation of an amount or concentration of a measurement target in the immunochromatography method is preferably performed by the method of the first aspect.

The method of the present invention may be for use in a heterogeneous immunoassay using a labeling substance. In this case, the labeling substance used in the heterogeneous immunoassay method using a labeling substance is preferably replaced with the labeling particles used in the method of the first aspect. In the heterogeneous immunoassay using a labeling substance, for example, when using an antibody containing a labeling substance (as an example, a radioisotope or an enzyme) or an antigen containing a labeling substance, the labeling substance among them may be replaced with the labeling particles used in the method of the first aspect. Irradiation of measurement light in the heterogeneous immunoassay method using a labeling substance is preferably performed by the method of the first aspect. Estimation of an amount or concentration of a measurement target in the heterogeneous immunoassay method using a labeling substance is preferably performed by the method of the first aspect.

The method of the present invention may be for use in an immunoassay selected from a competitive heterogeneous labeling method or non-competitive heterogeneous labeling method using a labeling substance. In this case, the labeling substance used in the immunoassay selected from a competitive heterogeneous labeling method or non-competitive heterogeneous labeling method using a labeling substance is preferably replaced with the labeling particles used in the method of the first aspect. In the immunoassay selected from a competitive heterogeneous labeling method or non-competitive heterogeneous labeling method using a labeling substance, for example, when using an antibody containing a labeling substance (as an example, a radioisotope or an enzyme) or an antigen containing a labeling substance, the labeling substance among them may be replaced with the labeling particles used in the method of the first aspect. Irradiation of measurement light in the immunoassay selected from a competitive heterogeneous labeling method or non-competitive heterogeneous labeling method using a labeling substance is preferably performed by the method of the first aspect. Estimation of an amount or concentration of a measurement target in the immunoassay selected from a competitive heterogeneous labeling method or non-competitive heterogeneous labeling method using a labeling substance is preferably performed by the method of the first aspect. Immunoassays can be divided into competitive type and non-competitive type based on differences in reaction mode. In the competitive type, a labeled antibody and an unlabeled antibody compete in reaction with an antigen. Alternatively, a labeled antigen and an unlabeled antigen compete in reaction with an antibody. On the other hand, the non-competitive type is a reaction mode without such competition as described above.

Further, immunoassays can be divided into homogeneous systems and heterogeneous systems. A homogeneous immunoassay is an immunoassay in which a reaction by reaction components such as antigens and antibodies is performed in one solution, and separation of a reaction component (B) bound by an antigen-antibody reaction and an unbound reaction component (F) after the reaction is not required. On the other hand, a heterogeneous system is an immunoassay requiring separation of B and F (B/F separation) after the reaction. The immunoassay method of the present

invention is preferably used in a heterogeneous immunoassay method. Examples of heterogeneous immunoassay methods include radioimmunoassay, enzyme immunoassay, fluorescence immunoassay, chemiluminescence immunoassay, immunoradiometric assay, and immunoenzymometric assay. In this case, the labeling substance (radioactive substance, enzyme, fluorescent substance, etc.) in each of the above measurement methods is preferably replaced with the labeling particles used in the method of the first aspect.

Further, the immunoassay method of the present invention is preferably used in a competitive heterogeneous labeling method or a non-competitive heterogeneous labeling method. Examples of the competitive heterogeneous labeling method include radioimmunoassay, enzyme immunoassay, ELISA method, fluorescence immunoassay, and chemiluminescence immunoassay. Examples of the non-competitive heterogeneous labeling method include immunoradiometric assay, immunoenzymometric assay, and immunochromatography method.

(Immunochromatography Method)

The immunochromatography method is a technique for detecting an antigen in a specimen using a test strip for immunochromatography in which a reagent containing an antibody causing an antigen-antibody reaction is applied to a measurement position. When detecting these, detection sensitivity is increased by binding a labeling substance to an antibody, an antigen, or a complex. The immunochromatography method is typically an analytical method utilizing a sandwich method. In the immunochromatography method utilizing a sandwich method, first, three types of antibodies recognizing different sites of a measurement target and a test strip are prepared, and for the first one, a labeled antibody using colloidal particles of metal and metal oxide as labeling particles is used in a conjugate pad of the test strip. Here, explanation is made with a generally widely used gold colloid labeled antibody. A second antigen-specific antibody (first capture antibody) is immobilized on a test line of a membrane of the test strip, and a third labeled antibody-specific antibody (second capture antibody) is immobilized on a control line downstream of the test line.

When a test solution containing a measurement target is added dropwise onto a sample pad, a measurement target-gold colloid labeled antibody complex is formed in the conjugate pad. When this complex passes through the test line, it is captured by the first capture antibody, a first capture antibody-measurement target-gold colloid label complex is formed, and a colored line appears on the test line. The gold colloid labeled antibody that did not form a complex with the measurement target is captured by the second capture antibody on the control line, a colored line appears on the control line, and it is judged that the inspection was performed correctly.

In the conventional method, detection and quantification of a measurement target are performed by measuring color development intensity of the colored line of the test line visually or by an optical measurement device. To replace this measurement method with the method of the present invention, light such as laser light may be irradiated onto the test line under conditions where Mie scattering occurs, intensity of light transmitted through the test strip (membrane) may be measured, and an amount or concentration of the measurement target may be estimated from this light intensity, instead of measuring the color development intensity of the colored line of the test line. Thereby, quantification of the measurement target becomes possible down to a lower concentration region than conventionally possible.

(Enzyme Immunoassay)

As one of enzyme immunoassays, the ELISA method is widely known. In the ELISA method, a measurement target (target protein or target antibody) is immobilized in each well of a well plate or the like, and if the measurement target substance is a protein, an enzyme-labeled antibody specifically binding to that protein is added. Thereafter, by adding a substrate that reacts with the enzyme, an enzymatic reaction occurs, so the measurement target substance is detected or quantified based on that enzyme activity. By replacing the labeling enzyme with the labeling particles used in the present invention, it is possible to detect or quantify the measurement target using the method of the present invention. In this case, addition of a substrate reacting with the enzyme becomes unnecessary. Depending on differences in labeling of a labeled antigen, besides enzyme immunoassay, radioimmunoassay, chemiluminescence immunoassay, fluorescence immunoassay, and the like are known. Although radioimmunoassay labels an antigen with a radioisotope, chemiluminescence immunoassay labels an antigen with a chemiluminescent substance, and fluorescence immunoassay labels an antigen with a fluorescent dye, differing from enzyme immunoassay, the measurement principles are common. To replace these measurement methods with the method of the present invention, the above labeling substance may be changed to the labeling particles used in the present invention, and measurement may be performed by the method of the present invention. Further, by installing a membrane or the like matching the bottom shape of a well of a well plate and immobilizing a measurement target on each membrane, a region where the measurement target labeled with labeling particles exists comes to have a thickness in a direction from a light source to a detector, and measurement with higher sensitivity becomes possible.

(Immunometric Assay Method)

In the immunometric assay method, an antigen (or antibody) as a measurement target and an excess amount of a labeled antibody (or labeled antigen) are reacted to form a complex, and then unreacted labeled antibody (or labeled antigen) is removed. Since the signal intensity of the labeling substance increases as the amount of the formed complex increases (as the measurement target increases), the amount or concentration of the measurement target can be estimated by obtaining the signal intensity when a sample containing the measurement target is added. In this case, by obtaining a relationship between an antigen (or antibody) whose concentration is already known and signal intensity as a calibration curve in advance, signal intensity when a sample containing a measurement target is added can be compared with the calibration curve, and the amount or concentration of the measurement target contained in the sample can be estimated. Note that depending on the type of label, variations exist such as an immunoradiometric assay method labeling an antibody (or antigen) with a radioisotope, and an immunoenzymometric assay method labeling an antibody (or antigen) with an enzyme. Note that in the case of labeling with an enzyme, a substrate is added to cause a substrate-enzyme reaction, and intensity of a signal such as luminescence or fluorescence emitted thereby is measured. To replace these measurement methods with the method of the present invention, the antibody (or antigen) labeled with the above radioisotope or enzyme may be changed to an antibody (or antigen) labeled with the labeling particles used in the present invention, and measurement may be performed by the method of the present invention.

(Sandwich Assay Method)

The sandwich assay method is known as a variation of an assay method utilizing the immunoradiometric assay method or the immunoenzymometric assay method, or as a variation of the ELISA method. The immunochromatography method is also a method utilizing the sandwich assay method. In the sandwich assay method, an antibody or antigen is immobilized in a well or the like, and a sample containing a measurement target (antigen or antibody) is added thereto and reacted. Thereafter, a labeled antibody or labeled antigen recognizing and binding to a measurement target site different from the immobilized antibody or antigen is added, and the measurement target is sandwiched between the antibody (or antigen) and the labeled antibody (or labeled antigen). Unreacted measurement target and labeled antibody (or labeled antigen) are removed. Since the labeled antibody or labeled antigen binds to the measurement target as the amount of the measurement target increases, the signal intensity of the label becomes stronger. In this case, by obtaining a relationship between a measurement target whose concentration is already known and signal intensity as a calibration curve in advance, signal intensity when a sample containing a measurement target whose concentration or amount is unknown is added can be compared with the calibration curve obtained in advance, and the concentration or amount of the measurement target contained in the sample can be estimated. To replace these measurement methods with the method of the present invention, the above labeled antibody (or labeled antigen) may be changed to an antibody (or antigen) labeled with the labeling particles used in the present invention, and measurement may be performed by the method of the present invention. Further, it is also possible to change a labeling substance used in a conventional immunoassay such as latex particles used in the latex agglutination method or latex agglutination inhibition method to the labeling particles used in the present invention, and perform measurement by the method of the present invention.

Further, by installing a membrane or the like matching the shape of the bottom surface of a well plate and immobilizing a measurement target on each membrane instead of each well, a region where the measurement target labeled with labeling particles exists comes to have a thickness in a direction from a light source to a detector, and measurement with higher sensitivity becomes possible.

(One Embodiment When Applied to Immunochromatography Method)

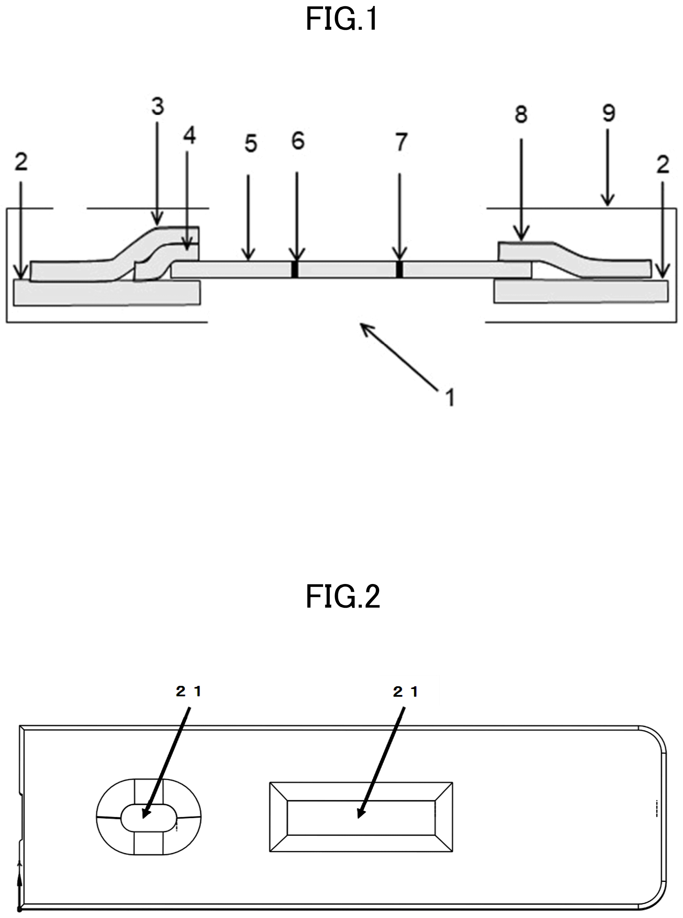

Hereinafter, with reference to Figs. 1 to 6, one embodiment when the method of the present invention is applied to an immunochromatography method which is one of immunoassay methods will be described.

Fig. 1 shows a schematic longitudinal cross-sectional view of a test strip 1 used in this embodiment. The test strip 1 is a lateral flow type test strip, is composed of a backing sheet 2, a sample pad 3, a conjugate pad 4, a membrane 5, an absorption pad 8, and the like, and has a structure accommodated in a housing case 9.

On the backing sheet, each member is arranged in the order of the sample pad 3, the conjugate pad 4, the membrane 5, and the absorption pad 8. The housing case 9 above the sample pad 3 has a hole, and a (liquid) sample capable of containing a measurement target is added dropwise onto the sample pad 3 from that hole. The conjugate pad 4 contains a plurality of labeling particles carrying an antibody capable of binding directly or indirectly to the measurement target, and when the sample is added dropwise onto the sample pad 3, the sample liquid develops to the conjugate pad 4, and the plurality of labeling particles carrying an antibody capable of binding directly or indirectly to the measurement target bind to the measurement target in the sample to form a complex. The sample liquid develops on the membrane, and the sample liquid containing the complex moves to a test line 6. A substance (antibody or the like) specifically binding to the

measurement target is arranged on the test line 6, and the measurement target (complex) labeled with the labeling particles is captured on the test line 6. On the other hand, labeling particles not bound to the measurement target pass through the test line together with the sample liquid and move to a control line 7. An antibody (IgG antibody or the like) against the antibody capable of binding directly or indirectly to the measurement target is arranged on the control line 7, and labeling particles not bound to the measurement target are captured here. The sample liquid finally develops to the absorption pad 8 and is absorbed.

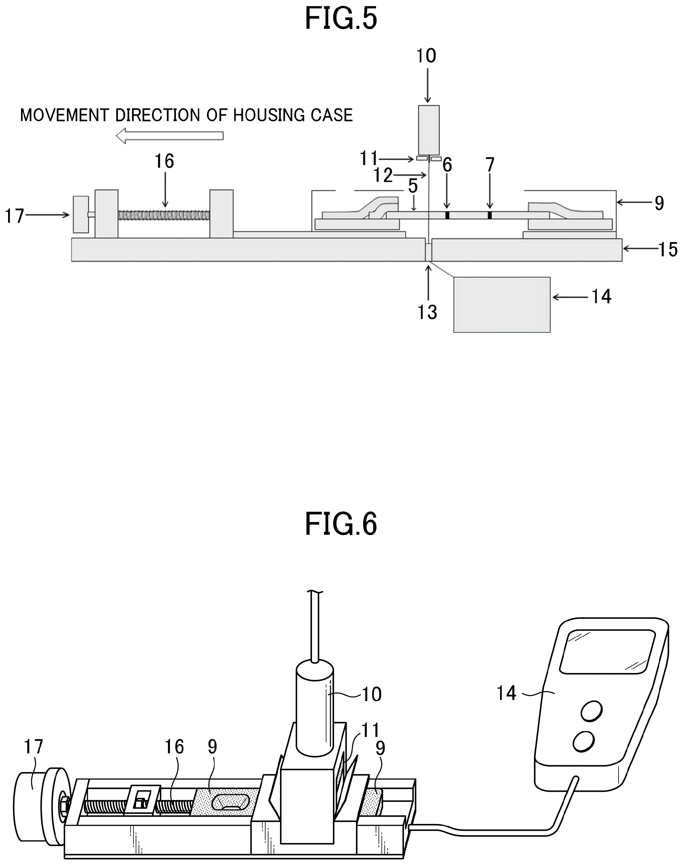

Fig. 5 is a schematic longitudinal cross-sectional view of an immunoassay apparatus of one embodiment that can be used in this embodiment, and shows a case where light 12 is irradiated from a light source 10 onto the test strip 1 held by a holding part (not shown) of the immunoassay apparatus. Fig. 6 is an external view (perspective view) of an immunoassay apparatus of one embodiment that can be used in this embodiment, wherein the test strip 1 is accommodated in a holding part configured such that a concave shape extends in one direction. Note that in Figs. 5 and 6, a slit part 11 is arranged between the light source and the test strip 1, but the slit part 11 may not be arranged. Further, the slit part 11 may be arranged not between the light source and the test strip 1 but between the test strip 1 and a light detection part 13, or may be arranged both between the light source 10 and the test strip 1 and between the test strip 1 and the light detection part 13. Further, in Fig. 6, in order to make the position where the slit part 11 is arranged easier to understand, it is illustrated with a cover beside the slit part 11 opened, but in use, this cover is closed to prevent entry of light from outside.

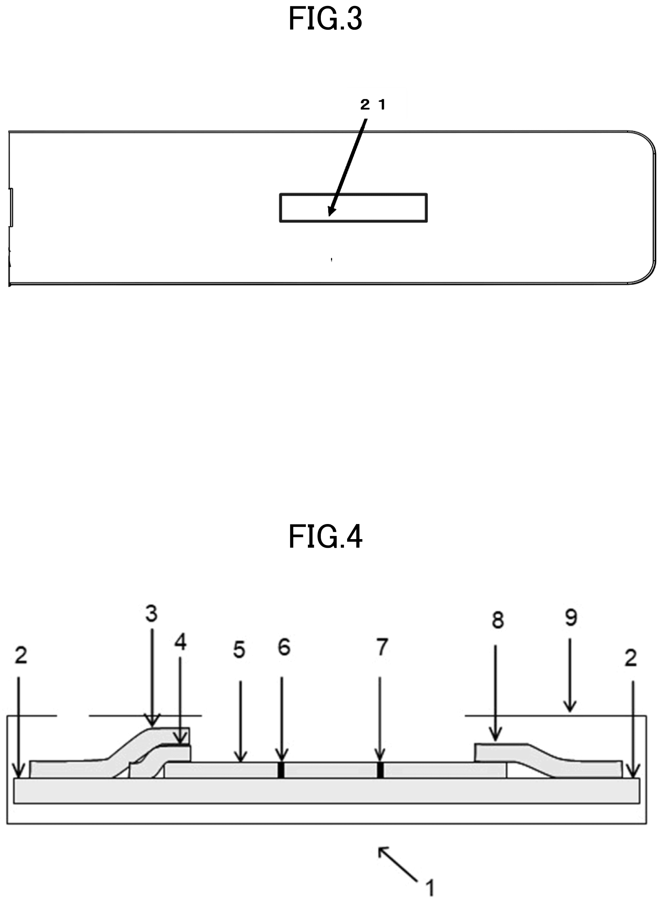

In the test strip 1 used in the present invention, unlike a conventional test strip (Fig. 4), the backing sheet 2 and the housing case 9 do not exist in a part of the back surface of the membrane 5 (see Figs. 1, 3, and 5). Thereby, light irradiated onto the membrane 5 and the test line 6 reaches the light detection part 13 on the side opposite to the light source 10 via the test strip 1, and the intensity of the reached light can be measured. The measured light intensity and the like can be displayed on a display part 14.

In this case, light 12 irradiated from the light source 10 may be irradiated not only onto the test line 6 but also onto the membrane 5 (region not containing labeling particles) other than the test line 6. When irradiating light onto the test line 6 and the membrane 5, light may be sequentially irradiated onto respective sites by sliding the test strip 1 by hand, but the test strip 1 may be slid in a lateral direction using a slide device 16. In this embodiment, the housing case 9 storing the test strip is installed on a pedestal connected to the slide device 16. In this case, a cavity is provided in the pedestal similarly to the housing case 9, therefore transmitted light can reach the light detection part 13 on the side opposite to the light source 10, and light intensity of the reached transmitted light can be measured. Furthermore, a handle for slide device 17 is arranged, and by rotating the handle 17, the pedestal of the slide device 16 connected to the handle 17 is slid in the left direction by a feed screw method. As an alternative method, the slide device 16 may comprise a member (for example, hook, magnet, spring, etc.) for connecting the test strip 1. In this case, by rotating the handle 17, a screw in the slide device 16 connected to the handle 17 rotates and extends in the right direction or contracts in the left direction, whereby the test strip 1 connected to the slide device 16 can also be slid in the right direction or the left direction.

Light 12 transmitted through the region containing labeling particles of the test line 6 is detected by the photodetector 13. The intensity of detected light is obtained by an estimation part. The light intensity may be displayed on the display part 14. The estimation part and the display part may be integrated. Similarly, light 12 transmitted through the membrane 5 is detected by the photodetector 13. The intensity of detected light is obtained by an estimation part. The light intensity may be displayed on the display part 14. The estimation part and the display part may be integrated. Next, a light attenuation rate is calculated from the light intensity of light transmitted through the test line and the light intensity of light transmitted through the membrane 5. The calculated light attenuation rate may be displayed on the display part 14.

Note that separately from the above measurement, it is preferable to obtain a calibration curve (or approximate line) showing a relationship between a concentration or amount of a measurement target and a light attenuation rate (or a relationship between a dilution rate of a measurement target and a light attenuation rate) using a sample in which the concentration or amount of the measurement target is known in advance.

By obtaining a calibration curve in advance, from comparison between a light attenuation rate when measured using a sample containing a measurement target of unknown concentration or amount and the calibration curve, the concentration or amount of the measurement target contained in said sample can be obtained. Thereby, the concentration or amount of the measurement target contained in the sample can be accurately estimated. The estimated concentration or amount of the measurement target contained in the sample may be displayed on the display part 14.

(One Embodiment When Applied to ELISA Method)

Next, with reference to Fig. 7, one embodiment when the method of the present invention is applied to the ELISA method which is another one of immunoassay methods will be described.

Fig. 7 is a schematic longitudinal cross-sectional view when light 12 is irradiated from a light source 10 onto a well plate 18 held by a holding part (not shown) of the immunoassay apparatus used in this embodiment.

The well plate 18 comprises one or a plurality of wells, preferably a plurality of wells (for example, 96 wells, etc.). Each well is preferably composed of a transparent material so that light can pass through the bottom of the well when light

12 is irradiated onto the well.

A sample containing a measurement target is added to a test sample well 20 in which an antibody for solid phase immobilization has been adsorbed on the surface of the well, and when the reaction is finished, excess liquid is discarded and the well is washed to bind the measurement target substance to the antibody for solid phase immobilization. Next, a sample containing a plurality of labeling particles carrying an antibody capable of binding directly or indirectly to the measurement target is added, reacted, and excess labeling particles are washed away to use as a test sample. Alternatively, a membrane or the like matching the bottom shape of the well is installed on the bottom surface, an antibody for solid phase immobilization is adsorbed to the membrane or the like, a sample containing a measurement target is added, and when the reaction is finished, excess liquid is discarded, the well with the installed membrane or the like is washed, and the measurement target substance is bound to the antibody for solid phase immobilization. Next, a sample containing a plurality of labeling particles carrying an antibody capable of binding directly or indirectly to the measurement target is added, reacted, and excess labeling particles are washed away. A region where the measurement target labeled with labeling particles exists comes to have a thickness in a direction from a light source to a detector, and measurement with higher sensitivity becomes possible.

For a control sample well 19, one in which an antibody for solid phase immobilization has been adsorbed on the surface of the well is used. Alternatively, when using a test sample in which a membrane or the like is installed on the bottom surface of the well, a membrane or the like matching the shape of the bottom surface of the well is installed, and one in which an antibody for solid phase immobilization has been adsorbed to the membrane is used as a test sample.

Light 12 is irradiated from the light source 10 onto the test sample well 20. The irradiated light passes through the test sample well and is detected by a light detection part 13 on the side opposite to the light source 10 across the test sample well 20. Thereby, intensity of reached light can be measured. The measured light intensity and the like may be displayed on a display part 14.

In this case, the well plate 18 is installed on a pedestal connected to a slide device 16. There, a cavity is provided in the pedestal, therefore transmitted light can reach the light detection part 13 on the side opposite to the light source 10, and light intensity of the reached transmitted light can be measured. Furthermore, a handle for slide device 17 is arranged, and by rotating the handle 17, the pedestal of the slide device 16 connected to the handle 17 is slid in the left direction by a feed screw method. As an alternative method, the slide device 16 may comprise a member (for example, hook, magnet, spring, etc.) for connecting the well plate 18. In this case, by rotating the handle 17, a screw in the slide device 16 connected to the handle 17 rotates and extends in the right direction or contracts in the left direction, whereby the well plate 18 connected to the slide device 16 can also be slid in the right direction or the left direction.

Light 12 transmitted through the region of the test sample well 20 is detected by the photodetector 13. The intensity of detected light is obtained by an estimation part. The light intensity may be displayed on the display part 14. The estimation part and the display part may be integrated. Similarly, light 12 transmitted through the control sample well 19 is detected by the photodetector 13. The intensity of detected light is obtained by an estimation part. The light intensity may be displayed on the display part 14. The estimation part and the display part may be integrated. Next, a light attenuation rate is calculated from the light intensity transmitted through the test sample well 20 and the light intensity transmitted through the control sample well 19. The calculated light attenuation rate may be displayed on the display part 14.

Note that separately from the above measurement, it is preferable to obtain a calibration curve (or approximate line) showing a relationship between a concentration or amount of a measurement target and a light attenuation rate (or a relationship between a dilution rate of a measurement target and a light attenuation rate) using a sample in which the concentration or amount of the measurement target is known in advance.

By obtaining a calibration curve in advance, from comparison between a light attenuation rate when measured using a sample containing a measurement target of unknown concentration or amount and the calibration curve, the concentration or amount of the measurement target contained in said sample can be obtained. Thereby, the concentration or amount of the measurement target contained in the sample can be accurately estimated. The estimated concentration or amount of the measurement target contained in the sample may be displayed on the display part 14.

In the above, embodiments in which the method of the first aspect (and the apparatus of the second aspect) is applied to the immunochromatography method and the ELISA method as representative examples of immunoassay methods have been described in detail. However, the method, apparatus, kit, and container or substrate of the present invention are not limited to application to these two immunoassay methods, and can be applied to various other immunoassay methods.

<Effects>

The present invention utilizes a principle that light scattering increases by combining a wavelength of light used as measurement light for immunoassay and a particle size of labeling particles carrying a measurement target under conditions where Mie scattering occurs. When measurement light passes through a region containing a measurement target, the measurement light hits labeling particles carrying the measurement target, and the light scattering amount is

exponentially increased by repeating scattering and transmission, so it has been found that transmitted light attenuates exponentially, and attenuation of light intensity can be measured even if labeling particles carrying a measurement target are at a low concentration.

The method of the present invention enables quantification of a measurement target down to a lower concentration region than conventionally possible by irradiating light onto labeling particles under conditions causing Mie scattering and detecting the transmitted light thereof.

The method of the present invention is a measurement method using an unprecedented new measurement principle of using light, preferably coherent laser light with aligned properties such as wavelength and phase as an input wavelength, utilizing a characteristic that directionality of light in a Mie scattering region increases by a combination of particle sizes of labeling particles, and enabling quantitative measurement of light attenuation amount in a dispersion medium, and is different from existing measurement principles utilizing reflected light. As a feature of this measurement method, it is a measurement method that not only achieves sensitivity that cannot be measured by the reflection method but also combines reproducibility important for quantitativeness.

This measurement method can be preferably used in a measurement system presenting a state where labeling particles having labeled a measurement target by an antigen-antibody reaction exist with a thickness not only in a two-dimensional direction but also in an optical axis direction in a measurement region (or medium). It is applicable to various media satisfying the above conditions, such as a membrane, a liquid phase, a gel phase, a solid phase, and the like as a medium.

Further, one embodiment applying the method of the first aspect to an immunochromatography method is a method completely different from a conventional immunochromatography method by a light reflection method, and has characteristics of rapidity and specificity of inspection, and further high sensitivity in a low concentration region of a measurement target. According to the present invention, a linear result is obtained down to a lower concentration region of a measurement target, and rapid quantitative inspection becomes possible in the immunochromatography method.

Conventionally, in the generally widely used light reflection method, reflected light obtained as a result of attenuation by two-dimensional (planar) absorption and scattering of particles existing on an optical axis with respect to incident light is measured. In contrast, the present invention solves the problem by not an absorption method measuring reflected light by particles near the surface on a test line two-dimensionally, but by applying a relationship between particle size and wavelength of incident light and a light effect of labeling particles, and three-dimensionally measuring an attenuation amount of detection light with respect to incident light by an effect of Mie scattering by all particles existing along the optical axis in a test line zone.

The method and apparatus of the present invention can be applied to various immunoassay methods as described above, and are not limited to the immunochromatography method or the ELISA method.

## Examples

Next, test examples related to the present invention will be described, but these do not limit the present invention.

### [Example 1]

<Measurement of Light Attenuation Rate of Transmitted Light under Mie Scattering Conditions>

As follows, each material used in Example 1 was prepared, a light attenuation rate of transmitted light when C-reactive protein (CRP) as a measurement target was diluted to various concentrations was obtained, and a relationship between the dilution rate and the light attenuation rate was investigated.

Note that when using the wavelength of 450 nm and the particle size of labeling particles of 454 nm of Example 1, $\alpha$=about 3.2, and the wavelength of measurement light is a wavelength generating Mie scattering by each of the labeling particles.

(1) Synthesis of resin particles

Labeling particles (platinum-resin composite particles, average particle size 454 nm) were synthesized according to the following procedure.

After dissolving Aliquat 336 [manufactured by Aldrich] (1.50 g) and polyethylene glycol methyl ether methacrylate (PEGMA, 10.00 g) in 300 g of pure water, 2-vinylpyridine (2-VP, 49.50 g) and divinylbenzene (DVB, 0.50 g) were added, and stirred at 30°C for 50 minutes and then at 60°C for 30 minutes under a nitrogen stream. After stirring, 2,2-azobis(2-methylpropionamidine) dihydrochloride (AIBA, 0.50 g) dissolved in 18.00 g of pure water was added dropwise, and stirred at 60°C for 3.5 hours to obtain resin particles with an average particle size of 430 nm. After precipitating by centrifugation (9000 rpm, 45 minutes) and removing supernatant, impurities were removed by dialysis treatment. Thereafter, concen-

tration adjustment was performed to obtain a 10 wt% resin particle dispersion liquid.

After adding 245 ml of pure water to the above resin particle dispersion liquid (90 ml), a 400 mM chloroplatinic acid aqueous solution (90 ml) was added, and after stirring at 30°C for 3 hours, it was left at room temperature for 24 hours. Thereafter, resin particles were precipitated by centrifugation (3100 rpm, 30 minutes), and supernatant was removed to remove excess chloroplatinic acid. Thereafter, concentration adjustment was performed to prepare a 5 wt% platinum ion adsorbed resin particle dispersion liquid.

Next, the 5 wt% platinum ion adsorbed resin particle dispersion liquid (55 ml) was added to 3825 ml of pure water, and while stirring at 3°C, a 132 mM dimethylamine borane aqueous solution (110 ml) was added dropwise, and then stirred at 3°C for 1 hour. Thereafter, by stirring at 25°C for 3 hours, a platinum-resin composite with an average particle size of 454 nm was obtained. The platinum-resin composite was precipitated by centrifugation (3100 rpm, 60 minutes), and after removing supernatant, it was purified by dialysis treatment to remove impurities. Thereafter, concentration adjustment was performed to obtain a 1 wt% platinum-resin composite dispersion liquid. The average particle size of formed platinum particles was 5 nm, and the supported amount of platinum was 37.7 wt%.

<Measurement of Average Particle Size of Metal Particles>

From an image obtained by observing a substrate prepared by adding the platinum-resin composite particle dispersion liquid dropwise onto a metallic mesh with a carbon support film with a field emission scanning electron microscope (FE-SEM; manufactured by Hitachi High-Technologies Corporation, SU-9000), an area average diameter of 100 randomly selected metal particles was measured and defined as an average particle size.

<Measurement of Average Particle Size of Resin Particles and Platinum-Resin Composite Particles>

Measurement was performed using a centrifugal sedimentation particle size distribution analyzer (LUMiSizer 610 manufactured by LUM GmbH). Measurement was performed in a state where particles were dispersed in water or a solution. The median diameter (d50) of a particle size distribution obtained by the centrifugal sedimentation particle size distribution analyzer was defined as the average particle size of labeling particles.

(2) Binding of labeling particles and antibody

After mixing 25 μg of anti-CRP antibody and 0.45 mL of 50 mM HEPES buffer (pH 7), 0.05 mL of the 1 wt% platinum-resin composite particle dispersion liquid (solvent is water) prepared in (1) above was added, and end-over-end mixing was performed at room temperature for 1 hour to obtain a labeled antibody dispersion liquid A containing anti-CRP antibody labeled with platinum-resin composite particles.

(3) Blocking step

The labeled antibody dispersion liquid A obtained in (2) was centrifuged at 3000 rpm for 5 minutes, and supernatant liquid was removed. Thereafter, 0.5 mL of HEPES buffer (pH 7) containing 1 wt% sodium caseinate was added to the sediment, and after ultrasonic dispersion, end-over-end mixing was further performed at room temperature for 1 hour to obtain a labeled antibody dispersion liquid B.

(4) Washing treatment

The labeled antibody dispersion liquid B was centrifuged at 3000 rpm for 5 minutes, and supernatant was removed. Thereafter, 0.5 mL of 5 mM Tris aqueous solution (pH 8.5) containing less than 0.1 wt% surfactant was added to the sediment, and ultrasonically dispersed. This operation was repeated 3 times to obtain a labeled antibody dispersion liquid C.

(5) Preparation of conjugate pad

The labeled antibody dispersion liquid C was centrifuged at 3000 rpm for 5 minutes, and supernatant was removed. Thereafter, 5 mM Tris aqueous solution (pH 8.5) containing 5 wt% sucrose and 2.5 wt% BSA was added to the sediment, and ultrasonically dispersed to obtain a labeled antibody dispersion liquid D. After uniformly impregnating a glass fiber non-woven fabric with the labeled antibody dispersion liquid D, drying was performed at 50°C for 1 hour to prepare a conjugate pad 4. At this time, the concentration of the labeled antibody dispersion liquid D and the application amount to the glass fiber non-woven fabric were adjusted so that the content of platinum-resin composite particles in the conjugate pad 4 would be 3 μg per test in the immunochromatography method evaluation described later.

(6) Preparation of test strip

As shown in Fig. 1, a test line 6 was drawn by applying an anti-CRP antibody solution with a concentration of 1 mg/mL to a nitrocellulose membrane 5 having a width of 25 mm and a thickness of 100 μm. Further, a control line 7 was drawn by applying an anti-mouse IgG antibody solution with a concentration of 0.5 mg/mL to the downstream side of the test line 6. After drying this nitrocellulose membrane 5 at 50°C for 1 hour, a backing sheet 2, a conjugate pad 4, a sample pad 3 (glass fiber non-woven fabric), and an absorption pad 8 (cotton non-woven fabric) were laminated as in the cross-sectional view of the test strip 1 shown in Fig. 1, with parts thereof removed so that light passes through. Finally, it was cut to a width of 3.5 mm to create a test strip 1, which was stored in a housing case 9.

As for the housing case 9, as shown in Figs. 2 and 3, an opening 21 was provided not only on the upper surface of the housing case but also on the lower surface of the housing case so that light transmitted through the membrane could reach the light detection part without being blocked.

(7) Preparation of developing solution

An aqueous solution (pH 7.5) containing 50 mM Tris, 150 mM NaCl, 1.0 wt% BSA, and 1.0 wt% surfactant Triton X-100 was prepared and used as a developing solution.

(8) Preparation of immunoassay apparatus (Immunochromato Reader)

The configuration of the Immunochromato Reader apparatus consists of a light source 10, a slit 11, a holding part for the light source 10 and the slit 11, a light detection part 13, a slide base 15, a slide device 16, a handle for slide device 17, and a light attenuation rate measurement result display part 14, as shown in Figs. 5 and 6.

The casing of the holding part for the light source 10 and the slit 11, the casing of the slide base 15, the casing of the slide device 16, and the handle for slide device 17 were produced by a 3D printer (manufactured by FLASHFORGE, product name Adventurer5M Pro).

Specifically, for the light source 10, a semiconductor laser (manufactured by CivilLaser, output 10 mW, wavelength 450 nm) beam-shaped into a line was used, and for the slit 11, in parallel to the test line 6 of the test strip (for example, when the long side direction is 3.5 mm and the short side direction is 1 mm), an opening of 2 mm in the long side direction of the test line 6 and 0.3 mm in the short side direction of the test line 6 was provided, adjusted so that laser light irradiated from the light source 10 is irradiated only onto the test line 6 on the membrane 5 of the test strip 1, and fixed by the holding part. The holding part was structured such that laser light 12 irradiated from the light source 10 passes through the slit 11, further passes through the membrane 5 of the test strip 1, and can reach the light detection part 13.

The slide base 15 was designed such that two bar-shaped convex portions were provided along the long side direction on the upper side surface of the casing of the slide base 15 so that the housing case 9 storing the test strip 1 could be moved while being held, and the housing case 9 storing the test strip 1 was held between the two convex portions and could move only in the long side direction.

For the light detection part 13, a photodiode matched to the light amount of the light source 10 was used, and a groove was provided and installed in the slide base 15 so that laser light 12 irradiated from the light source 10 and passed through the slit 11 could be received directly below. The holding part holding the light source 10 and the slit 11 was designed to be perpendicular to the slide base 15. The light detection part 13 was connected to the measurement result display part 14. For the measurement result display part 14, an illuminometer (manufactured by Zhangzhou Weihua Electronic, product name LX-1010B) was used.

The slide device 16 was designed using a feed screw mechanism so that it could slide in the long side direction with high accuracy while holding the housing case 9 as shown in Figs. 5 and 6. The slide device 16 was designed to be movable by turning the handle for slide device 17.

(9) Measurement and evaluation by immunochromatography method

Using the developing solution, specimen liquids of positive samples in which CRP antigen 3.12 mg/mL was diluted as shown in Tables 1 and 2 and a negative control (antigen-free) were prepared. 50 μL of specimen liquid was added dropwise onto the sample pad 3 of the test strip. Note that concentrations of CRP antigen (pg/mL) at each dilution rate are as in Tables 1 and 2.

Table 1

| Positive Sample | 1 | 2 | 3 | 4 | 5 | 6 | 7 |
|---|---|---|---|---|---|---|---|
| Dilution Rate | 1/10,000 | 1/40,000 | 1/160,000 | 1/320,000 | 1/640,000 | 1/1,280,000 | 1/2,560,000 |

(continued)

| Positive Sample | 1 | 2 | 3 | 4 | 5 | 6 | 7 |
|---|---|---|---|---|---|---|---|
| pg/mL | 312,000 | 78,000 | 19,500 | 9,750 | 4,875 | 2,437.50 | 1,218.75 |

Table 2

| Positive Sample | 8 | 9 | 10 | 11 | 12 | 13 | 14 |
|---|---|---|---|---|---|---|---|
| Dilution Rate | 1/5,120,000 | 1/10,240,000 | 1/20,480,000 | 1/40,960,000 | 1/81,920,000 | 1/163,840,000 | 1/327,680,000 |
| pg/mL | 609.38 | 304.69 | 152.34 | 76.17 | 38.09 | 19.04 | 9.52 |

After 30 minutes, light intensity of transmitted light of each of the membrane on the sample pad side (background) and the test line was measured by the Immunochromato Reader apparatus. A light attenuation rate (%) was obtained by subtracting the light intensity of the test line from the light intensity of the membrane and dividing by the light intensity of the membrane according to the following formula.

$$\text{Light attenuation rate (\%)}=(B-A)/B\times 100$$

A: Light intensity (unit: lx) detected by photodiode (light detection part) when light is irradiated onto test line.
B: Light intensity (unit: lx) detected by photodiode (light detection part) when light is irradiated onto membrane.

The result is shown in Fig. 8.

[Comparative Example 1]

<Color Development Intensity Measurement Using Reflected Light Which Is Conventional Method (Part 1)>

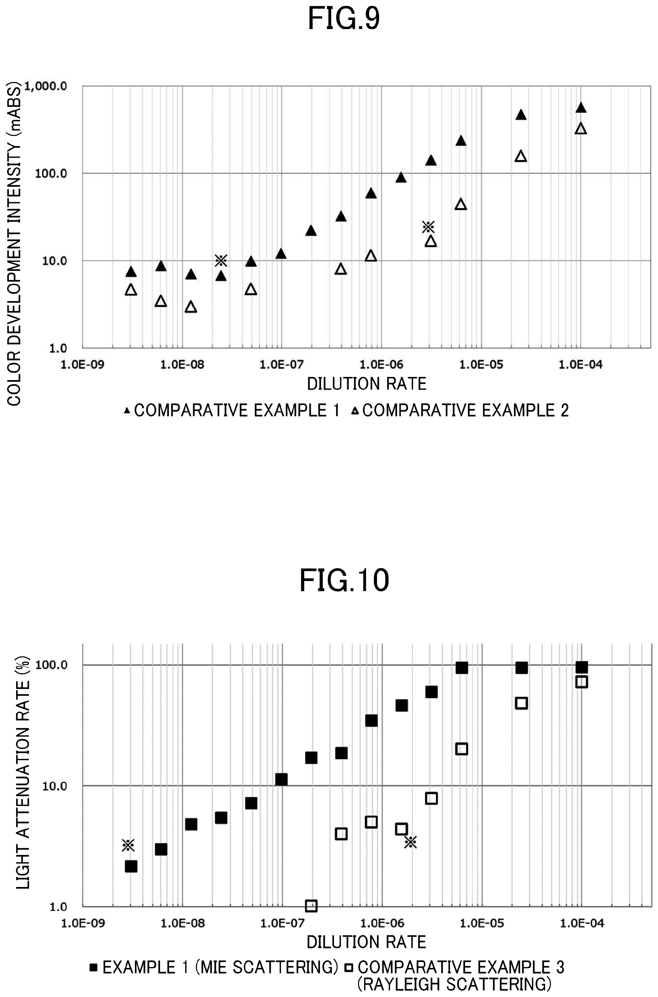

In Comparative Example 1, color development intensity measurement was performed utilizing reflected light which is a conventional method instead of transmitted light, and a relationship between dilution rate and color development intensity was investigated.
Specifically, evaluation by immunochromatography method was performed in the same manner as in Example 1 except that a test strip 1 in which the backing sheet 2 was not partially removed in (6) of Example 1 was produced as shown in Fig. 4, an opening was not provided at the lower part of the housing case 9 storing the test strip 1, C10066-10 which is a commercial product manufactured by Hamamatsu Photonics K.K. was used as an Immunochromato Reader in (8) and (9), and color development intensity (mABS) of reflected light instead of transmitted light was measured in the evaluation by immunochromatography method of (9).
The result is shown in Fig. 9.

[Comparative Example 2]

<Color Development Intensity Measurement Using Reflected Light Which Is Conventional Method (Part 2)>

In Comparative Example 2, a test similar to Comparative Example 1 was performed using gold colloid particles instead of platinum-resin composite particles. That is, similarly to Comparative Example 1, color development intensity measurement was performed utilizing reflected light which is a conventional method instead of transmitted light, and a relationship between dilution rate and color development intensity was investigated.
Specifically, evaluation by immunochromatography method was performed in the same manner as in Comparative Example 1 except that gold colloid (Tanaka Kikinzoku Kogyo, Au colloid solution-SC, particle size 30 nm) was used as labeling particles, 5 mM Tris aqueous solution (pH 9.5) was used as a binding buffer, and 5 mM Tris aqueous solution (pH 9.5) containing 1 wt% BSA was used as a blocking buffer.
The result is shown in Fig. 9.

[Comparative Example 3]

<Measurement of Light Attenuation Rate of Transmitted Light under Conditions Not Causing Mie Scattering>

In Comparative Example 3, attenuation rate of transmitted light was measured in the same manner as in Example 1 under conditions not causing Mie scattering (specifically, conditions causing Rayleigh scattering), and a relationship between dilution rate and color development intensity was investigated.
Specifically, evaluation by immunochromatography method was performed in the same manner as in Example 1 except using gold colloid (Tanaka Kikinzoku Kogyo, Au colloid solution-SC, particle size 30 nm) as labeling particles, 5 mM Tris aqueous solution (pH 9.5) as a binding buffer, and 5 mM Tris aqueous solution (pH 9.5) containing 1 wt% BSA as a blocking buffer.
The result is shown in Fig. 10.

<Results>

In Example 1 measuring the light attenuation rate of transmitted light under conditions causing Mie scattering,

linearity was obtained even when CRP was diluted to 1/327,680,000 times (Fig. 8).

In contrast, in the conventional color development intensity measurement of reflected light, linearity was obtained only up to a dilution rate of 1/40,960,000 times in Comparative Example 1 (Fig. 9). At a dilution rate lower than 1/40,960,000 times, a value almost equivalent to the negative control was shown, and a result that quantitative measurement in a low concentration region is difficult compared to Example 1 was obtained. Similarly, in Comparative Example 2, linearity was obtained only up to a dilution rate of 1/320,000 times (Fig. 9). At a dilution rate lower than 1/320,000 times, a value almost equivalent to the negative control was shown, and a result that quantitative measurement in a low concentration region is difficult compared to Example 1 was obtained. Note that marks * in Figs. 8 and 9 indicate the maximum dilution rate of CRP antigen at which linearity was obtained.

Further, even when measuring the light attenuation rate of transmitted light, under conditions of Comparative Example 3 where Mie scattering does not occur, linearity was obtained only up to a dilution rate of 1/640,000 times (Fig. 10). At a dilution rate lower than 1/640,000 times, a value almost equivalent to the negative control was shown, and a result that quantitative measurement in a low concentration region is difficult compared to Example 1 was obtained. Note that marks * in Fig. 10 indicate the maximum dilution rate of CRP antigen at which linearity was obtained.

Note that in Comparative Example 3, instead of preparing platinum-resin composite particles under conditions not causing Mie scattering, gold colloid particles generally widely used in immunochromatography method were used, but since the degree of change in light attenuation rate when changing dilution rate is determined by the relationship between particle size and wavelength of incident light, it is considered that similar results would be obtained even when using platinum-resin composite particles under conditions not causing Mie scattering.

The method using transmission of light of the present invention is, as described above, one in which light attenuation in the straight traveling direction increases exponentially according to transmission distance because Mie scattering generated by conditions of particle diameter and wavelength of incident light based on principles of photophysics becomes dominant and scatters light in all directions. The difference in metal material of particles does not affect the particle diameter at all, and does not affect Mie scattering at all. On the other hand, the difference in metal material of particles gives a change to particle color (light absorption), and gives a large influence to the conventional light reflection method mainly based on light absorption, but does not affect the method mainly based on Mie scattering of the present invention.

From the above, it could be concluded that measurement of light attenuation rate of transmitted light under conditions where Mie scattering occurs enables quantitative measurement in a lower concentration region compared to the case of measuring color development intensity of reflected light or the case of measuring under conditions where Mie scattering does not occur.

[Example 2]

<Measurement of Light Attenuation Rate at Various Wavelengths of Laser Light>

Mie scattering occurs depending on the relationship between particle size and wavelength of incident light. Therefore, it was verified whether reproducibility of light attenuation rate measurement can be obtained similarly even when the wavelength of light is changed within a range under conditions causing Mie scattering, not only at the specific wavelength used in Example 1. Note that particles with the same particle size as in Example 1 were used as the particle size of particles.

Two types of light sources (light source 1, light source 2) in which the wavelength of the semiconductor laser was changed were prepared, and evaluation by immunochromatography method was performed in the same manner as in Example 1 using each light source.

Light source 1: Semiconductor laser (manufactured by CivilLaser, output 10 mW, wavelength 520 nm)
Light source 2: Semiconductor laser (manufactured by CivilLaser, output 10 mW, wavelength 648 nm)

Note that when light source 1 was used, $\alpha$=about 2.7, and when light source 2 was used, $\alpha$=about 2.2, both of which are within the Mie scattering generation region.

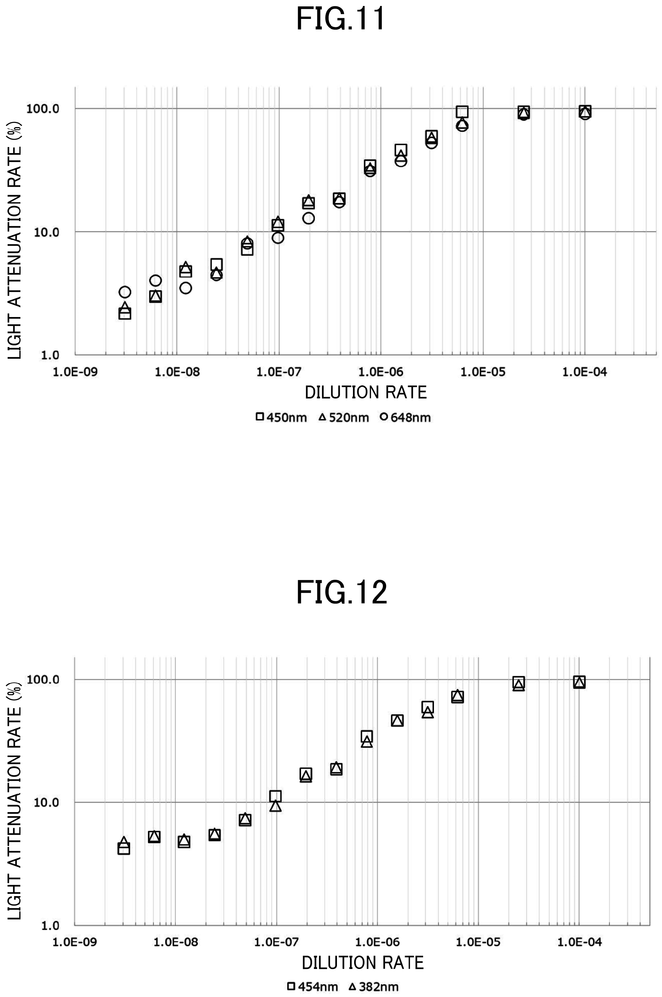

The result is shown in Fig. 11.

<Results>

As a result of light attenuation rate measurement using different wavelengths of 520 nm and 648 nm respectively, equivalent results with quantitativeness up to the same dilution rate as light attenuation rate measurement using the wavelength of 450 nm were obtained for 520 nm and 648 nm as well (Fig. 11). From this, it was confirmed that within a range of conditions where the relationship between particle size and wavelength of incident light causes Mie scattering,

equivalent results with quantitativeness can be obtained even when the wavelength is changed.

[Example 3]

<Measurement of Light Attenuation Rate at Various Particle Sizes>

As described above, Mie scattering occurs depending on the relationship between particle size and wavelength of incident light. Therefore, in Example 3, it was verified whether reproducibility of light attenuation rate measurement can be obtained similarly even when the particle size is changed within the range of Mie scattering region, not only at the specific particle size used in Example 1. Note that the same wavelength as in Example 1 was used as the light wavelength.

(1) Synthesis of labeling particles

Labeling particles 1 (platinum-resin composite particles, average particle size 454 nm) were produced according to the procedure of Example 1.

Labeling particles 2 (platinum-resin composite particles, average particle size 382 nm) were synthesized according to the following procedure.

Note that when labeling particles 1 were used, $\alpha$=about 3.2, and when labeling particles 2 were used, $\alpha$=about 2.7, both of which are within the Mie scattering generation region.

After dissolving Aliquat 336 [manufactured by Aldrich] (3.00 g) and polyethylene glycol methyl ether methacrylate (PEGMA, 10.00 g) in 300 g of pure water, 2-vinylpyridine (2-VP, 49.50 g) and divinylbenzene (DVB, 0.50 g) were added, and stirred at 30°C for 50 minutes and then at 60°C for 30 minutes under a nitrogen stream. After stirring, 2,2-azobis(2-methylpropionamidine) dihydrochloride (AIBA, 0.50 g) dissolved in 18.00 g of pure water was added dropwise, and stirred at 60°C for 3.5 hours to obtain resin particles with an average particle size of 370 nm. After precipitating by centrifugation (9000 rpm, 45 minutes) and removing supernatant, impurities were removed by dialysis treatment. Thereafter, concentration adjustment was performed to obtain a 10 wt% resin particle dispersion liquid.

After adding 245 ml of pure water to the above resin particle dispersion liquid (90 ml), a 400 mM chloroplatinic acid aqueous solution (90 ml) was added, and after stirring at 30°C for 3 hours, it was left at room temperature for 24 hours. Thereafter, resin particles were precipitated by centrifugation (3100 rpm, 30 minutes), and supernatant was removed to remove excess chloroplatinic acid. Thereafter, concentration adjustment was performed to prepare a 5 wt% platinum ion adsorbed resin particle dispersion liquid.

Next, the 5 wt% platinum ion adsorbed resin particle dispersion liquid (55 ml) was added to 3825 ml of pure water, and while stirring at 3°C, a 132 mM dimethylamine borane aqueous solution (110 ml) was added dropwise, and then stirred at 3°C for 1 hour. Thereafter, by stirring at 25°C for 3 hours, a platinum-resin composite with an average particle size of 382 nm was obtained. The platinum-resin composite was precipitated by centrifugation (3100 rpm, 60 minutes), and after removing supernatant, it was purified by dialysis treatment to remove impurities. Thereafter, concentration adjustment was performed to obtain a 1 wt% platinum-resin composite dispersion liquid. The average particle size of formed platinum particles was 5 nm, and the supported amount of platinum was 38.5 wt%.

(2) Binding of labeling particles and antibody

After mixing 25 $\mu$g of anti-CRP antibody and 0.45 mL of 50 mM HEPES buffer (pH 7), 0.05 mL of any of the 1 wt% platinum-resin composite particle dispersion liquids (solvent is water) prepared in (1) above was added, and end-over-end mixing was performed at room temperature for 1 hour to obtain a labeled antibody dispersion liquid A containing anti-CRP antibody labeled with platinum-resin composite particles.

Except for that, evaluation by immunochromatography method was performed in the same manner as in Example 1. The result is shown in Fig. 12.

<Results>

As a result of light attenuation rate measurement using different particle sizes of platinum-resin composite particles of 382 nm and 454 nm respectively, results with quantitativeness down to a low concentration region were obtained for both particle sizes (Fig. 12). From this, it was confirmed that within a range where the relationship between particle size and wavelength of incident light meets the conditions for Mie scattering, equivalent results with quantitativeness can be obtained even when the particle size is changed.

[Industrial Applicability]

According to the present invention, quantification is possible even if a measurement target in a sample has a low concentration. Therefore, the present invention is extremely useful industrially.

Reference Signs List

1... Test strip
2... Backing sheet
3... Sample pad
4... Conjugate pad
5... Membrane
6... Test line
7... Control line
8... Absorption pad
9... Housing case
10... Light source
11... Slit
12... Light (laser light)
13... Light detection part
14... Display part
15... Slide base
16... Slide device
17... Handle for slide device
18... Well plate
19... Control sample well
20... Test sample well
21... Opening

## Claims

**1.** An immunoassay method comprising

a step of irradiating measurement light onto a container or substrate containing: a sample containing a measurement target; and a plurality of labeling particles carrying an antibody capable of binding directly or indirectly to the measurement target, after contact between the sample containing the measurement target and the plurality of labeling particles carrying an antibody capable of binding directly or indirectly to the measurement target, wherein the measurement light is irradiated into a region containing the labeling particles of the container or substrate; and
a step of detecting light (transmitted light) that has passed through the region among the irradiated measurement light,
wherein a wavelength of the irradiated measurement light is a wavelength that generates Mie scattering by each of the plurality of labeling particles, and
the step of irradiating the measurement light includes
irradiating measurement light into the region containing the plurality of labeling particles of the container or substrate (hereinafter referred to as a first region), and
irradiating measurement light into a second region outside the first region.

**2.** The immunoassay method according to claim 1, wherein the wavelength that generates Mie scattering by each of the plurality of labeling particles is a wavelength satisfying $2<\alpha<10$ (where $\alpha=\pi\times$particle size of labeling particle (nm)/light wavelength (nm)).

**3.** The immunoassay method according to claim 1, further comprising a step of separating, after the contact and before irradiating the measurement light, the antibody carried on a labeling particle that is bound directly or indirectly to the measurement target from the antibody carried on a labeling particle that is not bound to the measurement target.

4. The immunoassay method according to claim 1, further comprising a step of obtaining a concentration or amount of the measurement target from light intensity of the detected transmitted light.

5. The immunoassay method according to claim 1, comprising measuring a light intensity 1 of measurement light that has passed through the first region, and a light intensity 2 of measurement light that has passed through the second region.

6. The immunoassay method according to claim 5, further comprising a step of determining a light attenuation rate by the following formula:

Light attenuation rate=(Light intensity 2-Light intensity 1)×100/Light intensity 2.

7. The immunoassay method according to claim 6, further comprising a step of obtaining a calibration curve or an approximate line showing a relationship between the concentration or amount of the measurement target and the light attenuation rate, using a sample in which the concentration or amount of the measurement target is known in advance.

8. The immunoassay method according to claim 6, further comprising a step of determining a light attenuation rate using a sample containing a measurement target of unknown concentration or amount, comparing the light attenuation rate with the calibration curve or approximate line obtained in advance in claim 7, and estimating the concentration or amount of the measurement target contained in the sample.

9. The immunoassay method according to claim 1, wherein the light is coherent measurement light.

10. The immunoassay method according to claim 9, wherein the coherent measurement light is laser light.

11. The immunoassay method according to claim 1, wherein the labeling particles contain a metal.

12. An immunoassay apparatus, comprising:

a holding part for holding a container or substrate containing a plurality of labeling particles carrying an antibody capable of binding directly or indirectly to a measurement target, inside or on the apparatus;
a light source disposed above the holding part for irradiating measurement light into a region containing the plurality of labeling particles of the container or substrate held by the holding part; and
a light detection part disposed on a side opposite to the light source across the container or substrate, detecting transmitted light irradiated from the light source and having passed through the region containing the labeling particles of the container or substrate,
wherein a wavelength of measurement light irradiated by the light source is a wavelength that generates Mie scattering by each of the plurality of labeling particles,
wherein the immunoassay apparatus is configured to irradiate measurement light into each of the region containing the plurality of labeling particles of the container or substrate (first region) and a region outside the first region of the container or substrate (second region).

13. The immunoassay apparatus according to claim 12, for use in the method according to claim 1.

14. The immunoassay apparatus according to claim 12, wherein the wavelength that generates Mie scattering by each of the plurality of labeling particles is a wavelength satisfying $2<\alpha<10$ (where $\alpha=\pi\times$particle size of labeling particle (nm)/light wavelength (nm)).

15. The immunoassay apparatus according to claim 12, further comprising the container or substrate containing the plurality of labeling particles carrying an antibody capable of binding directly or indirectly to the measurement target.

16. A kit for immunoassay, comprising

the apparatus according to claim 12; and
a container or substrate containing a plurality of labeling particles carrying an antibody capable of binding directly or indirectly to a measurement target.

**17.** A kit for immunoassay, comprising

the apparatus according to claim 12; and
a container or substrate capable of allowing disposition of a plurality of labeling particles carrying an antibody capable of binding directly or indirectly to a measurement target.

**18.** The kit for immunoassay according to claim 17, further comprising a plurality of labeling particles carrying an antibody capable of binding directly or indirectly to a measurement target.

**19.** A container or substrate containing a plurality of labeling particles carrying an antibody capable of binding directly or indirectly to a measurement target, for use in the method according to claim 1, or for use in the apparatus according to claim 12, or for use in the kit according to claim 16, or of use in the kit according to claim 17.

FIG.1

3
4
5
6
7
8
9
2
2
1

FIG.2

2 1
2 1

FIG.3

2 1

FIG.4

3
4
5
6
7
8
9
2
2
1

FIG.5

10
MOVEMENT DIRECTION OF HOUSING CASE
16
11
12
6
7
5
17
9
15
13
14


FIG.6

10
14
11
17
16
9
9

# FIG.7

10
MOVEMENT DIRECTION OF WELL PLATE
19
20
16
11
12
17
18
15
13
14

# FIG.8

LIGHT ATTENUATION RATE (%)
100.0
10.0
1.0
※
1.0E-09
1.0E-08
1.0E-07
1.0E-06
1.0E-05
1.0E-04
DILUTION RATE
■EXAMPLE 1

# FIG.9

COLOR DEVELOPMENT INTENSITY (mABS)
1,000.0
100.0
10.0
1.0
1.0E-09
1.0E-08
1.0E-07
1.0E-06
1.0E-05
1.0E-04
DILUTION RATE
※
COMPARATIVE EXAMPLE 1
COMPARATIVE EXAMPLE 2

# FIG.10

LIGHT ATTENUATION RATE (%)
100.0
10.0
1.0
1.0E-09
1.0E-08
1.0E-07
1.0E-06
1.0E-05
1.0E-04
DILUTION RATE
※
EXAMPLE 1 (MIE SCATTERING)
COMPARATIVE EXAMPLE 3 (RAYLEIGH SCATTERING)

# FIG.11

100.0
10.0
1.0
LIGHT ATTENUATION RATE (%)
1.0E-09
1.0E-08
1.0E-07
1.0E-06
1.0E-05
1.0E-04
DILUTION RATE
□450nm △520nm ○648nm

# FIG.12

100.0
10.0
1.0
LIGHT ATTENUATION RATE (%)
1.0E-09
1.0E-08
1.0E-07
1.0E-06
1.0E-05
1.0E-04
DILUTION RATE
□454nm △382nm

TRANSLATION

## INTERNATIONAL SEARCH REPORT

International application No. **PCT/JP2025/036374**

**A. CLASSIFICATION OF SUBJECT MATTER**

***G01N 21/47***(2006.01)i; ***G01N 33/543***(2006.01)i
FI: G01N21/47 Z; G01N33/543 521

According to International Patent Classification (IPC) or to both national classification and IPC

**B. FIELDS SEARCHED**

Minimum documentation searched (classification system followed by classification symbols)

G01N21/47; G01N33/543

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2017-015520 A (HITACHI HIGH-TECHNOLOGIES CORPORATION) 19 January 2017 (2017-01-19) | 1-19 |
| A | JP 2015-078933 A (FUJIKURA LTD.) 23 April 2015 (2015-04-23) | 1-19 |
| A | JP 2003-504642 A (WILLIAM MARSH RICE UNIVERSITY) 04 February 2003 (2003-02-04) | 1-19 |
| A | JP 08-043390 A (KDK CORP.) 16 February 1996 (1996-02-16) | 1-19 |
| A | JP 07-146295 A (KDK CORP.) 06 June 1995 (1995-06-06) | 1-19 |
| A | WO 2022/085790 A1 (TAUNS LABORATORIES, INC.) 28 April 2022 (2022-04-28) | 1-19 |
| A | JP 2007-120976 A (MATSUSHITA ELECTRIC INDUSTRIAL CO., LTD.) 17 May 2007 (2007-05-17) | 1-19 |
| A | US 2021/0171781 A1 (CHEMTREAT, INC.) 10 June 2021 (2021-06-10) | 1-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 December 2025** | **23 December 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

TRANSLATION

INTERNATIONAL SEARCH REPORT

International application No.
PCT/JP2025/036374

C. DOCUMENTS CONSIDERED TO BE RELEVANT

| Category* | Citation of document, with indication, where appropriate, of the relevant passages | Relevant to claim No. |
|---|---|---|
| A | US 9678005 B1 (ARIZONA BOARD OF REGENTS ON BEHALF OF THE UNIVERSITY OF ARIZONA) 13 June 2017 (2017-06-13) | 1-19 |
| A | CN 118169787 A (SHENZHEN INSTITUTES OF ADVANCED TECHNOLOGY) 11 June 2024 (2024-06-11) | 1-19 |

Form PCT/ISA/210 (second sheet) (July 2022)

TRANSLATION

**INTERNATIONAL SEARCH REPORT**
**Information on patent family members**

International application No.
**PCT/JP2025/036374**

| Patent document cited in search report | Publication date (day/month/year) | Patent family member(s) | Publication date (day/month/year) |
|---|---|---|---|
| JP 2017-015520 A | 19 January 2017 | WO 2017/002436 A1 | |
| JP 2015-078933 A | 23 April 2015 | (Family: none) | |
| JP 2003-504642 A | 04 February 2003 | JP 2010-230679 A | |
| | | US 6699724 B1 | |
| | | US 2005/0130324 A1 | |
| | | WO 2001/006257 A1 | |
| | | EP 1210600 A1 | |
| | | CA 2377722 A1 | |
| JP 08-043390 A | 16 February 1996 | US 5750410 A | |
| | | US 6887430 B1 | |
| | | EP 786665 A1 | |
| JP 07-146295 A | 06 June 1995 | US 5607643 A | |
| | | US 5869346 A | |
| | | EP 654670 A2 | |
| WO 2022/085790 A1 | 28 April 2022 | US 2023/0393139 A1 | |
| | | EP 4235177 A1 | |
| | | CN 116420066 A | |
| | | KR 10-2023-0089573 A | |
| JP 2007-120976 A | 17 May 2007 | (Family: none) | |
| US 2021/0171781 A1 | 10 June 2021 | WO 2019/113005 A1 | |
| | | EP 3692185 A1 | |
| | | EP 4474360 A2 | |
| US 9678005 B1 | 13 June 2017 | US 2010/0136521 A1 | |
| | | US 9562855 B1 | |
| | | US 2010/0136610 A1 | |
| | | WO 2010/065669 A1 | |
| | | WO 2010/065698 A1 | |
| CN 118169787 A | 11 June 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6381642 B
- JP 2006250787 A
- JP 3436982 B
- WO 2017010391 A